# EUROPEAN PATENT APPLICATION

(11) **EP 4 830 723 A1**
(43) Date of publication of application: **28.10.2026**
(21) Application number: 24906592.1
(22) Date of filing: 23.12.2024
(51) Int. Cl.: A61K 45/00, A61K 31/7088, A61P 1/16, A61P 3/06

(54) **NEW TARGET FOR TREATING METABOLISM-ASSOCIATED FATTY LIVER DISEASE, REGULATORY MOLECULE THEREOF, AND USE THEREOF**

(30) Priority: 22.12.2023 CN 202311790463
(71) Applicant: Shanghai Pentogen Biotechnology Co., Ltd., Shanghai 200120 (CN)
(72) Inventor: TONG, Xuemei, CHONGQING ROAD Huangpu District, Shanghai 200025 (CN); TONG, Lingfeng, CHONGQING ROAD Huangpu District, Shanghai 200025 (CN); LIU, Junling, CHONGQING ROAD Huangpu District, Shanghai 200025 (CN); CHEN, Zhangbing, CHONGQING ROAD Huangpu District, Shanghai 200025 (CN); LI, Yakui, CHONGQING ROAD Huangpu District, Shanghai 200025 (CN); ZHU, Yemin, CHONGQING ROAD Huangpu District, Shanghai 200025 (CN); LI, Bin, Chongqing Road, Huangpu District Shanghai 200025 (CN); WANG, Guanzhen, Chongqing Road, Huangpu District Shanghai 200025 (CN); LIANG, Rui, Chongqing Road, Huangpu District Shanghai 200025 (CN)
(74) Representative: Gille Hrabal Partnerschaftsgesellschaft mbB Patentanwälte
(86) International application number: PCT/CN2024/141546
(87) International publication number: WO 2025/131122

(57) **Abstract**

The present invention provides a new target for treating metabolism-associated fatty liver disease, a regulatory molecule thereof, and use thereof. For the first time, the present invention discloses a novel target gene closely associated with the alleviation or treatment of metabolism-associated fatty liver disease and a signaling pathway associated with upstream and downstream regulation thereof. The present invention also discloses a new mechanism in which the signaling pathway is involved in regulating metabolism-associated fatty liver disease. The present invention further discloses a preferred medicament for the alleviation or treatment.

## Description

This application claims priority to patent application No. CN202311790463.8, filed on December 22, 2023; the entire contents of which are incorporated herein.

### TECHNICAL FIELD

The present disclosure belongs to the field of biomedicine, and more specifically, the present disclosure relates to a new target for treating metabolism-associated fatty liver disease, regulatory molecule thereof, and use thereof.

### BACKGROUND

Liver diseases can lead to diseases such as alcohol-associated liver disease (ALD), nonalcoholic fatty liver disease (NAFLD), and the like. These liver diseases can further induce liver tumors.

Fatty liver refers to a pathological change characterized by excessive accumulation of lipid within hepatocytes due to various causes. Fatty liver is generally more commonly seen in patients caused by disorders of lipid metabolism in hepatocytes resulting from various causes, these patients may experience symptoms such as right upper abdominal distension, loss of appetite, general weakness, listlessness, nausea, and the like; patients with severe disease may develop jaundice.

Nonalcoholic fatty liver disease (NAFLD) is characterized by hepatic steatosis. It is the most common cause of chronic liver disease, leading to many chronic liver diseases, such as liver fibrosis, cirrhosis, and liver failure, and is also a risk factor for primary liver cancer. Liver tumors caused by nonalcoholic steatohepatitis (NASH), a type of NAFLD, are showing an increasing trend.

Nonalcoholic fatty liver disease is qualitatively different from simple lipid accumulation in adipose tissue or lipid accumulation in other organs/tissues. The liver is an important site for lipid synthesis and metabolism in the body, while adipose tissue is more inclined to store excess lipids. The liver can perform de novo lipogenesis and lipolysis in response to systemic lipid balance. The liver converts excessive free fatty acids into triglycerides, which are then transferred to other tissues, such as adipose tissue, for storage. Dysfunction of the liver's lipid metabolism is an important trigger for the development of metabolic diseases (obesity, diabetes, hyperlipidemia, and the like) in the body.

In the past few decades, changes in lifestyle and dietary habits have promoted the epidemic of obesity and NAFLD. Currently, the growth rate of NAFLD patients is alarming. As liver diseases such as NAFLD become a major health problem, this field urgently needs more in-depth research and analysis to explore effective drug intervention methods. Revealing the mechanisms of occurrence and development of liver diseases such as NAFLD, and discovering potential therapeutic targets, is an urgent priority.

### SUMMARY

The object of the present disclosure is to provide a new target for treating metabolism-associated fatty liver disease (MAFLD), regulatory molecule thereof, and use thereof.

In the first aspect of the present disclosure, there is provided use of a downregulator of TKT or an (insulin-)InsR-C/EBPα-TKT signaling pathway comprising TKT in the preparation of a pharmaceutical composition for alleviating or treating metabolism-associated fatty liver disease.

In another preferred embodiment, the InsR-C/EBPα-TKT signaling pathway comprises: TKT gene/protein, C/EBPα gene/protein, InsR gene/protein; preferably also comprises the upstream and downstream regulatory genes/proteins or chemical molecules of TKT gene/protein, C/EBPα gene/protein, and InsR gene/protein.

In another preferred embodiment, the downregulator comprises a downregulator selected from: a downregulator of TKT, a downregulator of the C/EBPα-TKT interaction (mutual regulation; preferably including the transcriptional regulation of TKT by C/EBP), a downregulator of the InsR-C/EBPα interaction (mutual regulation).

In another preferred embodiment, the downregulator of TKT includes (but is not limited to): an agent that silences, knocks down, or knocks out the TKT gene, or an agent that inhibits TKT protein activity; more preferably, the downregulator of TKT includes: an interfering molecule that specifically interferes with TKT gene expression (such as siRNA, shRNA, miRNA, antisense nucleotide, etc.), a CRISPR gene editing reagent targeting the TKT gene, a homologous recombination reagent, or a site-directed mutagenesis reagent, wherein the reagent causes TKT to undergo a loss-of-function mutation.

In another preferred embodiment, the downregulator of the C/EBPα-TKT interaction includes (but is not limited to): an agent that weakens the binding of C/EBPα to the TKT promoter, an agent that silences, knocks down, or knocks out the C/EBPα gene, or an agent that inhibits C/EBPα protein activity; more preferably, the downregulator of the C/EBPα-TKT interaction includes: an agent that reduces the phosphorylation level of C/EBPα protein, an interfering molecule that specifically interferes with C/EBPα gene expression (such as siRNA, shRNA, miRNA, antisense nucleotide, etc.), a CRISPR gene editing reagent targeting the C/EBPα gene, a homologous recombination reagent, or a site-directed mutagenesis reagent, wherein the reagent causes C/EBPα to undergo a loss-of-function mutation.

In another preferred embodiment, the downregulator of the InsR-C/EBPα interaction includes (but is not limited to): an agent that silences, knocks down, or knocks out the InsR gene, an agent that inhibits InsR protein activity, or an agent that weakens insulin binding to InsR; more preferably, the downregulator of the InsR-C/EBPα interaction includes: an interfering molecule that specifically interferes with InsR gene expression (such as siRNA, shRNA, miRNA, antisense nucleotide, etc.), a CRISPR gene editing reagent targeting the InsR gene, a homologous recombination reagent, or a site-directed mutagenesis reagent, wherein the reagent causes InsR to undergo a loss-of-function mutation.

In another preferred embodiment, the mutual regulation comprises regulation at the transcriptional level.

In another preferred embodiment, the downregulator is an interfering reagent that silences the TKT gene; preferably, the interfering reagent is an siRNA; preferably, the siRNA is an siRNA having a nucleotide sequence (sense strand, with the corresponding antisense strand having a protective base at the 3' end) as shown in any one of SEQ ID NO: 1 to SEQ ID NO: 50 and SEQ ID NO: 53 to SEQ ID NO: 167; more preferably, the siRNA is an siRNA having a nucleotide sequence as shown in any one of SEQ ID NO: 37 (No. 1115), SEQ ID NO: 25 (No. 773), SEQ ID NO: 50 (siTKT16), SEQ ID NO: 49, SEQ ID NO: 61, SEQ ID NO: 63, SEQ ID NO: 73, SEQ ID NO: 74, SEQ ID NO: 78, SEQ ID NO: 88, or SEQ ID NO: 130.

In another preferred embodiment, the downregulator is a homologous recombination reagent that knocks down or knocks out the TKT gene, which causes the gene coding to be terminated.

In another preferred embodiment, the downregulator is a homologous recombination reagent that knocks down or knocks out the InsR gene, which causes the gene coding to be terminated.

In another preferred embodiment, the siRNA is formulated as an siRNA formulation for hepatocyte-targeted delivery; preferably, the siRNA is linked to a targeting ligand that binds to asialoglycoprotein receptor (ASGPR) (covalently bound, forming a conjugate).

In another preferred embodiment, the targeting ligand comprises: N-acetylgalactosamine (GalNAc), N-trifluoroacetylgalactosamine, N-propionylgalactosamine, N-n-butyrylgalactosamine or N-isobutyrylgalactosamine.

In another preferred embodiment, the metabolism-associated fatty liver disease comprises: nonalcoholic fatty liver disease (NAFLD), nonalcoholic steatohepatitis (NASH).

In another preferred embodiment, the downregulator of TKT or the (insulin-)InsR-C/EBPα-TKT signaling pathway comprising TKT increases inosine levels, enhances mitochondrial function, thereby improving hepatic lipid metabolism, alleviating or treating metabolism-associated fatty liver disease; preferably, the downregulator: prevents R5P derived from inosine from entering glycolysis, promotes the synthesis of inosine from glucose-derived R5P, elevates intracellular inosine levels, thereby promoting the inosine-PKA-CREB pathway, enhancing mitochondrial function, improving hepatic lipid metabolism, alleviating or treating metabolism-associated fatty liver disease; preferably, after promoting the inosine-PKA-CREB pathway, it further comprises: activating the PKA-CREB-ChoKβ axis, increasing phosphatidylcholine (PC) synthesis, thereby enhancing mitochondrial function; preferably, the increasing phosphatidylcholine (PC) synthesis is mediated by the CDP-choline pathway.

In another preferred embodiment, the metabolism-associated fatty liver disease is a metabolism-associated fatty liver disease with high expression of TKT in the liver (hepatocytes).

In another preferred embodiment, the "high expression" is "high expression" in the statistical meaning, for example, the average TKT expression in patients with "metabolism-associated fatty liver disease with high expression of TKT" is significantly higher by 10% or 20% or more, preferably higher by 30% or 50% or more, more preferably higher by 80% or 100% or more, compared with the average TKT expression in healthy individuals (or a statistically sufficient population).

In another aspect of the present disclosure, there is provided an siRNA or an siRNA formulation for alleviating or treating metabolism-associated fatty liver disease, wherein the siRNA comprises an siRNA having a nucleotide sequence as shown in any one of SEQ ID NO: 1 to SEQ ID NO: 50, and SEQ ID NO: 53 to SEQ ID NO: 167; preferably, the siRNA is an siRNA having a nucleotide sequence as shown in any one of SEQ ID NO: 37 (No. 1115), SEQ ID NO: 25 (No. 773), SEQ ID NO: 50 (siTKT16), SEQ ID NO: 49 (siTKT10), SEQ ID NO: 61, SEQ ID NO: 63, SEQ ID NO: 73, SEQ ID NO: 74, SEQ ID NO: 78, SEQ ID NO: 88, or SEQ ID NO: 130.

In another preferred embodiment, the siRNA formulation is a stable siRNA formulation for hepatocyte-targeted delivery.

In another preferred embodiment, the siRNA is linked to a targeting ligand that binds to asialoglycoprotein receptor (ASGPR) (covalently bound, forming a conjugate); the targeting ligand comprises: N-acetylgalactosamine (GalNAc), N-trifluoroacetylgalactosamine, N-propionylgalactosamine, N-n-butyrylgalactosamine or N-isobutyrylgalactosamine.

In the siRNA, the nucleotides in the sense strand and the antisense strand comprise modifications selected from the group consisting of: 2'-methoxy modification and 2'-fluoro modification; or, between adjacent nucleotides or between a nucleotide and a targeting ligand, they comprise a phosphorothioate linkage.

In another preferred embodiment, in the sense strand of the siRNA, from the 5' end, there are phosphorothioate linkages between the 1st and 2nd nucleotides and between the 2nd and 3rd nucleotides; in the corresponding antisense strand of the siRNA (with 2 protective bases at the 3' end), from the 5' end, there is a phosphorothioate linkage between the 1st and 2nd nucleotides; and from the 3' end, there are phosphorothioate linkages between the 1st and 2nd nucleotides and between the 2nd and 3rd nucleotides.

In another preferred embodiment, in the sense strand of the siRNA, from the 5' end, the nucleotides at positions 1, 2, 3, 4, 6, and 10-19 are 2'-methoxy modified; in the corresponding antisense strand (with 2 protective bases at the 3' end) of the siRNA, from the 5' end, the nucleotides at positions 1, 3, 4, 5, 7, 10-13, 15, and 17-21 are 2'-methoxy modified.

In another preferred embodiment, in the sense strand of the siRNA, from the 5' end, the nucleotides at positions 5, 7, 8, and 9 are 2'-fluoro modified; in the corresponding antisense strand (with 2 protective bases at the 3' end) of the siRNA, the nucleotides at positions 2, 6, 8, 9, 14, and 16 are 2'-fluoro modified.

In another aspect of the present disclosure, there is provided a pharmaceutical composition or kit for alleviating or treating metabolism-associated fatty liver disease, comprising the siRNA or siRNA formulation for alleviating or treating metabolism-associated fatty liver disease, and a pharmaceutically acceptable carrier or excipient.

In another aspect of the present disclosure, there is provided use of the InsR-C/EBPα-TKT signaling pathway in screening a substance for alleviating or treating metabolism-associated fatty liver disease.

In another preferred embodiment, the InsR-C/EBPα-TKT signaling pathway is a signaling pathway in hepatocytes.

In another aspect of the present disclosure, there is provided a method for screening a substance for alleviating or treating metabolism-associated fatty liver disease, comprising: (1) contacting a candidate substance with a system comprising the InsR-C/EBPα-TKT signaling pathway; (2) screening for a substance that downregulates the InsR-C/EBPα-TKT signaling pathway, wherein the substance is a substance (including a potential substance) useful for alleviating or treating metabolism-associated fatty liver disease; wherein the downregulating comprises: downregulating TKT, downregulating the C/EBPα-TKT interaction, and downregulating the (insulin/)InsR-C/EBPα interaction.

In another preferred embodiment, the step (1) comprises: adding a candidate substance to a system comprising the InsR-C/EBPα-TKT signaling pathway.

In another preferred embodiment, the step (2) comprises: detecting changes in each protein or an encoding gene thereof in the InsR-C/EBPα-TKT signaling pathway, and comparing with a control group, wherein the control group is a system comprising the InsR-C/EBPα-TKT signaling pathway without adding the candidate substance; if the candidate substance downregulates TKT, downregulates the C/EBPα-TKT interaction, or downregulates the (insulin/)InsR-C/EBPα interaction, then the candidate substance is a substance useful for alleviating or treating metabolism-associated fatty liver disease.

In another preferred embodiment, the system comprising the InsR-C/EBPα-TKT signaling pathway is selected from: a cell (culture) system, a subcellular (culture) system, a tissue (culture) system, or an animal system.

In another preferred embodiment, the downregulating may also be referred to as inhibiting, which is a statistically significant inhibiting or downregulating. For example, compared with a control or baseline, the inhibiting or downregulating is 10% or 20% or more, preferably 40% or 50% or more, and more preferably 80% or 100% or more.

In another preferred embodiment, the candidate substance comprises (but is not limited to): regulatory molecules designed against the InsR-C/EBPα-TKT signaling pathway, or its pathway proteins, or its upstream or downstream proteins or genes (such as but not limited to upregulators, interfering molecules, nucleic acid inhibitors, binding molecules (such as antibodies or ligands)), CRISPR constructs, small molecule compounds, and compounds from a compound library.

In another aspect of the present disclosure, there is provided use of a hepatic TKT protein or an encoding gene thereof in the preparation of a diagnostic reagent, wherein the diagnostic reagent is used for diagnosing or prognosing metabolism-associated fatty liver disease; preferably, the diagnosing or prognosing comprises: determining the occurrence or progression of metabolism-associated fatty liver disease, or determining whether it is suitable for applying a treatment regimen that uses "a downregulator of TKT or the (insulin-)InsR-C/EBPα-TKT signaling pathway comprising TKT" for treatment, based on the expression status of the hepatic TKT protein; if the TKT protein is highly expressed, then the treatment regimen is applicable.

In another aspect of the present disclosure, there is provided use of a reagent that specifically recognizes a TKT protein or an encoding gene thereof in the preparation of a diagnostic reagent or a diagnostic kit for diagnosing or prognosing metabolism-associated fatty liver disease; preferably, the diagnosing or prognosing comprises: determining the occurrence or progression of metabolism-associated fatty liver disease, or determining whether it is suitable for applying a treatment regimen that uses "a downregulator of TKT or the (insulin-)InsR-C/EBPα-TKT signaling pathway comprising TKT" for treatment, based on the expression status of the TKT protein; if the TKT protein is highly expressed, then the treatment regimen is applicable.

In another preferred embodiment, the diagnostic reagent comprises a diagnostic reagent selected from: a primer that specifically amplifies the gene encoding the TKT protein; a probe that specifically recognizes the gene encoding the TKT protein or its transcript; or an antibody that specifically binds to the TKT protein.

In another aspect of the present disclosure, there is provided a kit for diagnosing or prognosing metabolism-associated fatty liver disease, wherein the kit comprises: a diagnostic reagent that detects the expression status or expression level of a TKT protein or its encoding gene.

In another preferred embodiment, the kit further comprises: a nucleic acid extraction reagent, a polymerase chain reaction reagent, a Western blot reagent, and/or an enzyme-linked immunosorbent assay reagent.

Other aspects of the present disclosure, due to the disclosure herein, are apparent to those skilled in the art.

### BRIEF DESCRIPTION OF THE DRAWINGS

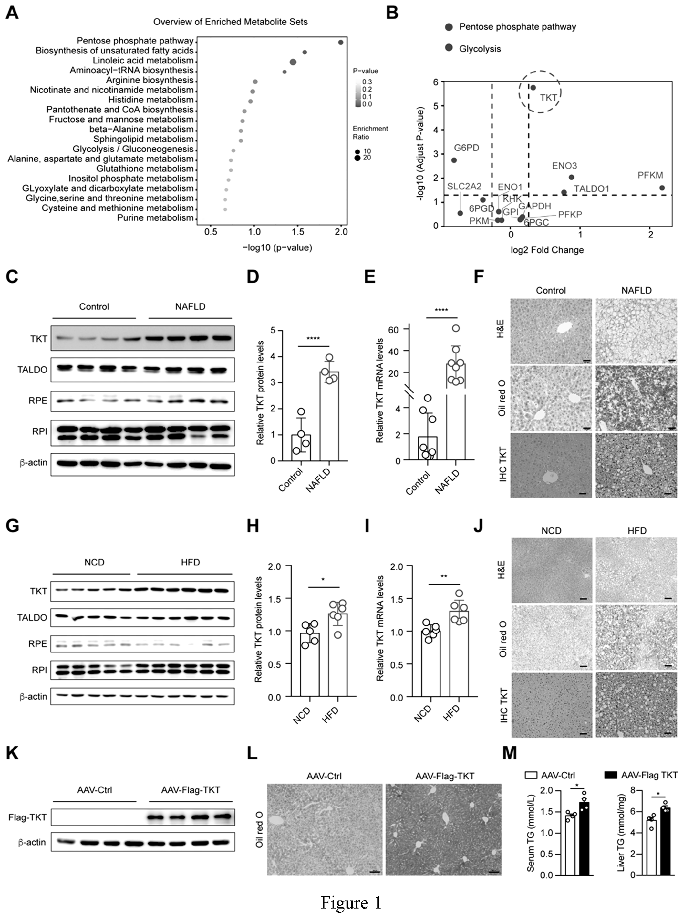

Figure 1. Analysis of the expression characteristics of TKT in liver tissue.
   (A) Differential metabolic pathway enrichment plot of healthy controls and NAFLD patients.
   (B) Differences in liver protein levels between NAFLD patients and healthy controls are displayed using a volcano plot.
   (C-E) Expression levels of non-oxidative pentose phosphate pathway (PPP) metabolic enzymes (C), TKT protein (D) and mRNA (E) in the livers of healthy controls and NAFLD patients (n = 4 in C and D, n = 8 in E).
   (F) H&E, Oil Red O, and TKT immunohistochemical staining of healthy controls and NAFLD patients (n = 4).
   (G-I) The expression levels of metabolic enzymes in the non-oxidative pentose phosphate pathway (PPP) (G), quantification of TKT protein (H) and mRNA (I) levels in the liver of wild-type mice after feeding with a normal chow diet (NCD) or a high-fat diet (HFD) for 90 days (n = 5 for NCD, n = 6 for HFD).
   (J) H&E, Oil Red O, and TKT immunohistochemical staining of liver tissue of wild-type mice after feeding with a normal chow diet (NCD) or a high-fat diet (HFD) for 90 days (n = 5 in NCD, n = 6 in HFD).
   (K) Hepatic TKT protein expression level of mice after tail-vein injection of AAV-Flag-TKT adenovirus for 10 weeks (n = 4).
   (L) Oil Red O staining for liver tissue of mice after tail-vein injection of AAV-Flag-TKT adenovirus and feeding with a HFD for 10 weeks (n = 4).
   (M) Levels of serum and liver triglycerides (TG) of mice after tail-vein injection of AAV-Flag-TKT adenovirus and feeding with a HFD for 10 weeks (n = 4).
   For all: *p < 0.05, **p < 0.01, ***p < 0.005, ****p < 0.001. Data are represented as mean ± SEM. Two-tailed t test was applied for D, E, H, I, and M.

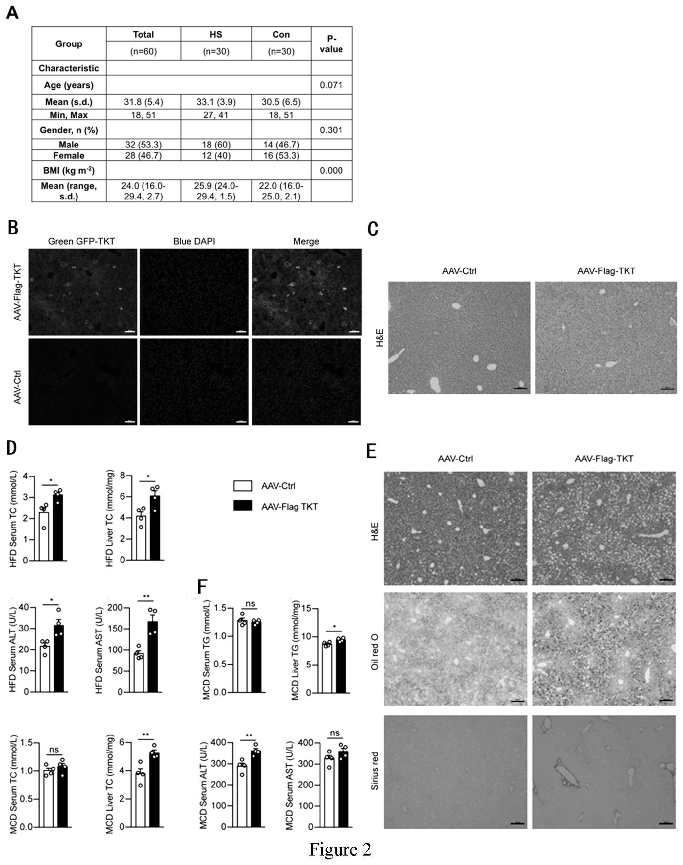

Figure 2. Supplementary analysis of the expression characteristics of TKT in liver tissue.
   (A) Clinical sample information corresponding to the liver tissue used in the experiment. HS: Hepatic Steatosis.
   (B) Liver fluorescence images of wild-type mice after tail vein injection of AAV-Flag-TKT adenovirus for 10 weeks (n = 3).
   (C and D) H&E staining of the liver tissue, serum and liver TC levels, serum ALT and AST levels of wild-type mice after tail vein injection of AAV-Flag-TKT adenovirus and feeding with a HFD for 10 weeks (n = 4).
   (E and F) H&E, Oil Red O, and Sirius Red staining of the liver tissue, serum and liver TG and TC levels, serum ALT and AST levels of wild-type mice after tail vein injection of AAV-Flag-TKT adenovirus and feeding with an MCD for 4 weeks (n = 4).
   For all: *p < 0.05, **p < 0.01, ***p < 0.005, ****p < 0.001. Data are represented as mean ± SEM. Two-tailed t-test was applied for D and F.

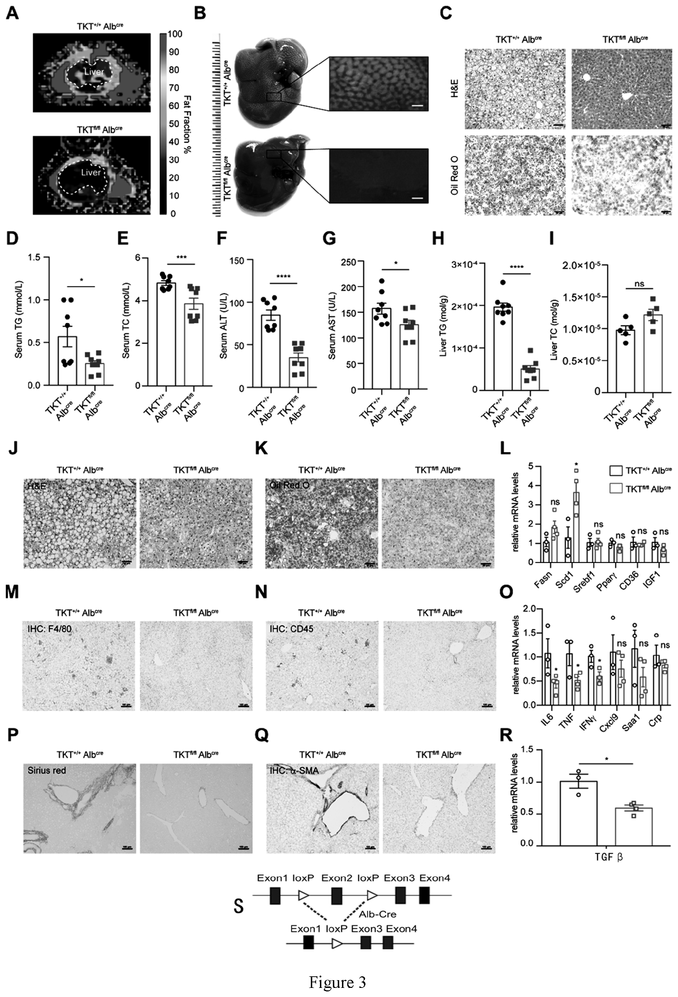

Figure 3. Effect of hepatocyte-specific TKT knockout on disease progression.
   (A-I) Phenotypic analysis of wild-type and liver-specific TKT-deficient mice after feeding with a HFD for 90 days, including (A) nuclear magnetic resonance imaging of liver lipid content, (B) liver appearance, (C) H&E and Oil Red O staining, (D) serum TG levels, (E) serum TC levels, (F) serum ALT levels, (G) serum AST levels, (H) liver TG and (I) liver TC levels (n = 5 to 8).
   (J-R) Phenotypic analysis of wild-type and liver-specific TKT-deficient mice after feeding with an MCD for 4 weeks, including (J) H&E staining, (K) Oil Red O staining, (L) mRNA levels of lipid synthesis-related genes, immunohistochemical staining of (M) F4/80 and (N) CD45, (O) mRNA levels of inflammation-related genes, (P) Sirius Red staining, (Q) immunohistochemical staining of alpha-smooth muscle actin (α-SMA), and (R) TGFβ mRNA expression levels (n = 3 to 4).
   (S) Construction strategy of liver-specific TKT knockout mice.
   For all: *p < 0.05, **p < 0.01, ***p < 0.005, ****p < 0.001. Data are represented as mean ± SEM. Two-tailed t test was applied for D, E, F, G, H, I, L, O, and R.

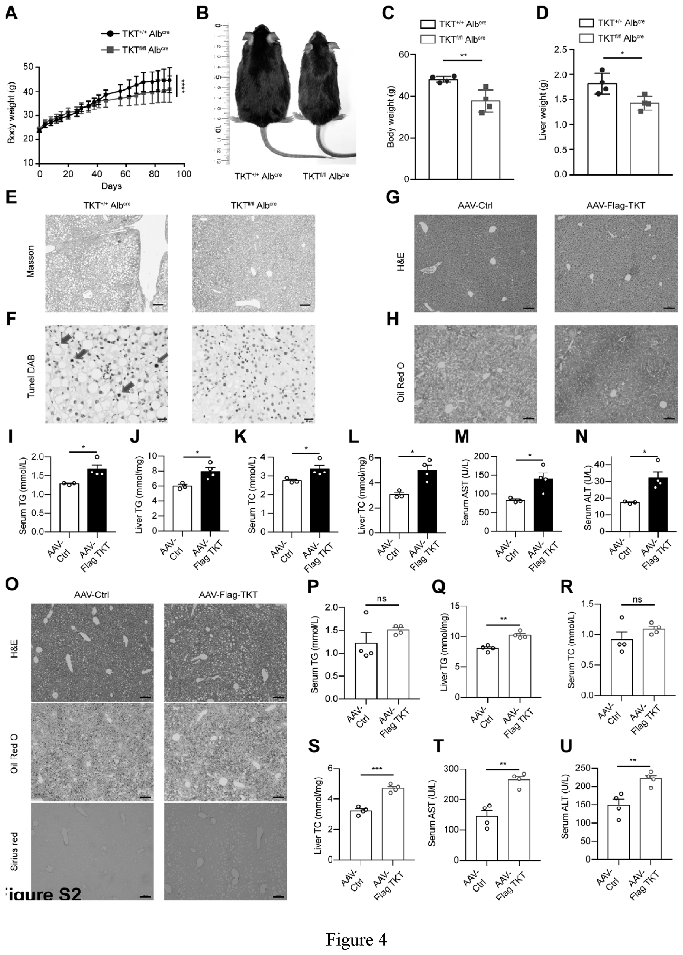

Figure 4. Effect of regulating TKT in the liver on disease progression.
   (A-D) (A) Body weight change, (B) body shape images, (C) body weight, and (D) liver weight of wild-type and liver-specific TKT-deficient mice after feeding with a HFD for 90 days (n = 4).
   (E and F) Liver pathological features of wild-type and liver-specific TKT-deficient mice after feeding with an MCD for 28 days, including (E) Sirius Red staining and (F) TUNEL staining (n = 4).
   (G-N) Phenotypic analysis of TKT^{fl/fl}Alb^{cre} mice after tail vein injection of AAV-Flag-TKT adenovirus and feeding with a HFD for 10 weeks, including (G) H&E staining, (H) Oil Red O staining, (I) serum TG, (J) liver TG, (K) serum TC, (L) liver TC, (M) serum AST, and (N) serum ALT levels (n = 3 in control group, n = 4 in AAV-Flag-TKT group).
   (O-U) Phenotypic analysis of TKT^{fl/fl} Alb^{cre} mice after tail vein injection of AAV-Flag-TKT adenovirus and feeding with an MCD for 3 weeks, including (O) H&E staining, Oil Red O and Sirius Red staining, and (P) serum TC, (Q) liver TC, (R) serum TG, (S) liver TG, (T) serum AST, and (U) serum ALT levels (n = 4).
   For all data: *p<0.05, **p<0.01, ***p<0.005, ****p<0.001. Data are presented as mean ± SEM. Two-tailed t-test was applied for C, D, I, J, K, L, M, N, P, Q, R, S, T, and U.

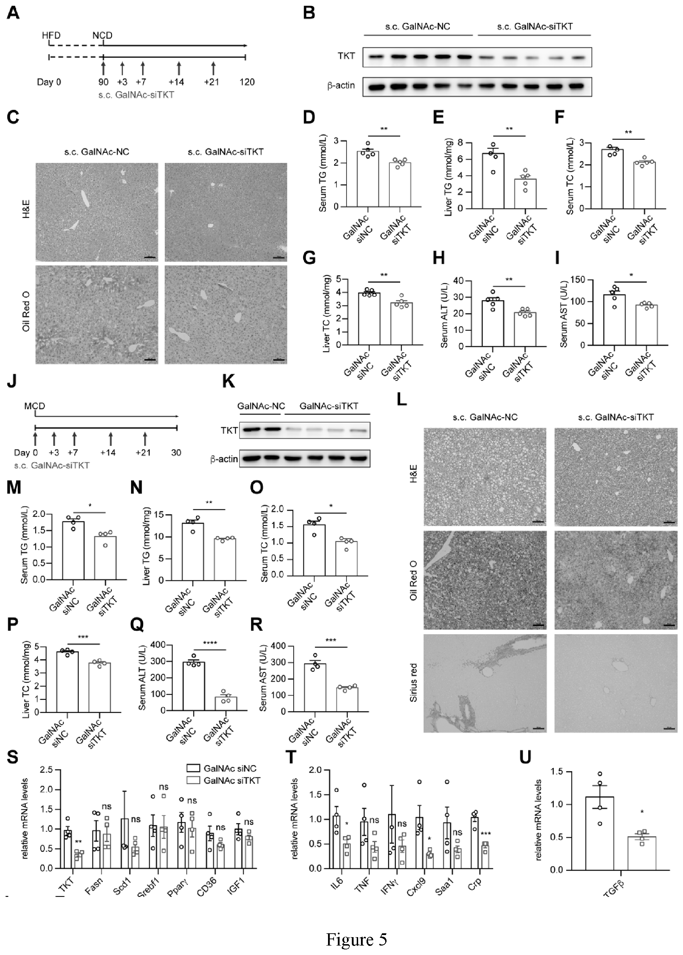

Figure 5. GalNAc-siRNA targeting hepatic TKT and the effect thereof.
   (A) Injection strategy of GalNAc-siTKT for treating NAFL. Wild-type male mice at 8 weeks of age were fed a high-fat diet for 90 days, followed by subcutaneous injection of GalNAc-siTKT at a dose of 10 mg/kg in the back. The injection was repeated 5 times on days 90 + 0, 3, 7, 14, and 21, respectively. The analysis was performed on day 120.
   (B) Expression level of hepatic TKT protein of NAFL mice after GalNAc-siTKT treatment (n = 5).
   (C-I) Phenotypic analysis of NAFL mice after GalNAc-siTKT treatment, including (C) H&E and Oil Red O staining, (D) serum TG, (E) liver TG, (F) serum TC, (G) liver TC, (H) serum ALT, and (I) AST levels (n = 5).
   (J) Injection strategy of GalNAc-siTKT for treating NASH. Wild-type male mice at 8 weeks of age were subcutaneously injected with 10 mg/kg GalNAc-siTKT on the back at the beginning of the MCD feeding. The injection was repeated 5 times on days 0, 3, 7, 14, and 21, respectively. The analysis was performed on day 30.
   (K) Expression level of hepatic TKT protein of NASH mice after GalNAc-siTKT intervention (n = 2 for NC, n = 4 for siTKT).
   (L-U) Phenotypic analysis of NASH mice after GalNAc-siTKT intervention, including (L) H&E, Oil Red O, Sirius Red staining, and (M) serum TG, (N) liver TG, (O) serum TC, (P) liver TC, (Q) serum ALT, and (R) serum AST levels, and (S) mRNA levels of lipogenic genes, (T) inflammation-related genes, and (U) fibrosis-related genes (n = 4).
   For all: *p < 0.05, **p < 0.01, ***p < 0.005, ****p < 0.001. Data are represented as mean ± SEM. Two-tailed t test was applied for D, E, F, G, H, I, M, N, O, P, Q, R, S, T, and U.

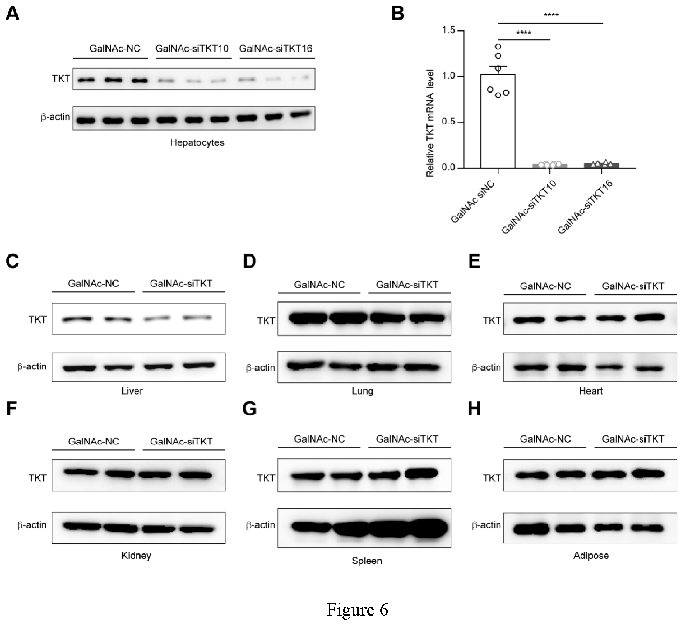

Figure 6. GalNAc-siRNA specifically targets and reduces hepatic TKT protein levels in vivo in mouse.
   (A and B) (A) Protein and (B) mRNA levels of TKT in primary hepatocytes from wild-type mice treated with GalNAc-siTKT without liposome encapsulation.
   (C-H) TKT protein expression levels in (C) liver, (D) lung, (E) heart, (F) kidney, (G) spleen, and (H) adipose tissue of wild-type mice 72 hours after subcutaneous injection of 10 mg/kg GalNAc-siTKT.
   For all: *p < 0.05, **p < 0.01, ***p < 0.005, ****p < 0.001. Data are represented as mean ± SEM. Two-tailed t-test was applied for B.

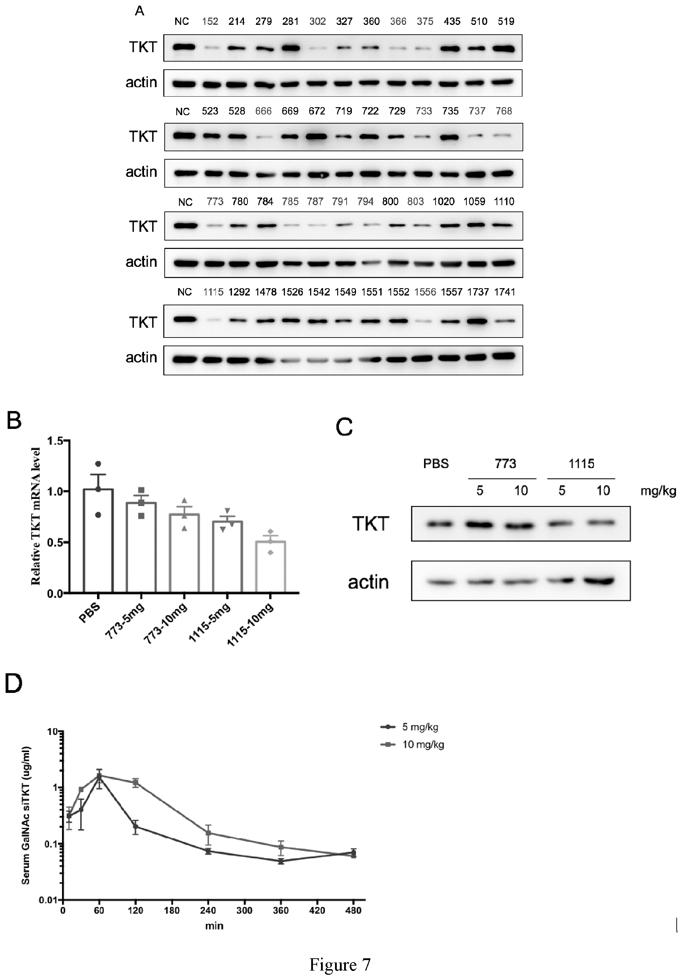

Figure 7. Screening and efficacy analysis of human, mouse, and monkey homologous sequences.
   (A) Knockdown efficacy of a total of 49 siRNAs targeting the homologous regions of the human, mouse, and monkey TKT gene in mouse primary hepatocytes.
   (B-C) TKT knockdown efficacy in the liver of wild-type mice 7 days after subcutaneous injection of GalNAc-siTKT 773 and GalNAc-siTKT 1115, (B) mRNA level and (C) protein level.
   (D) Changes in blood concentration of GalNAc-siTKT 1115 after subcutaneous injection in wild-type mice.

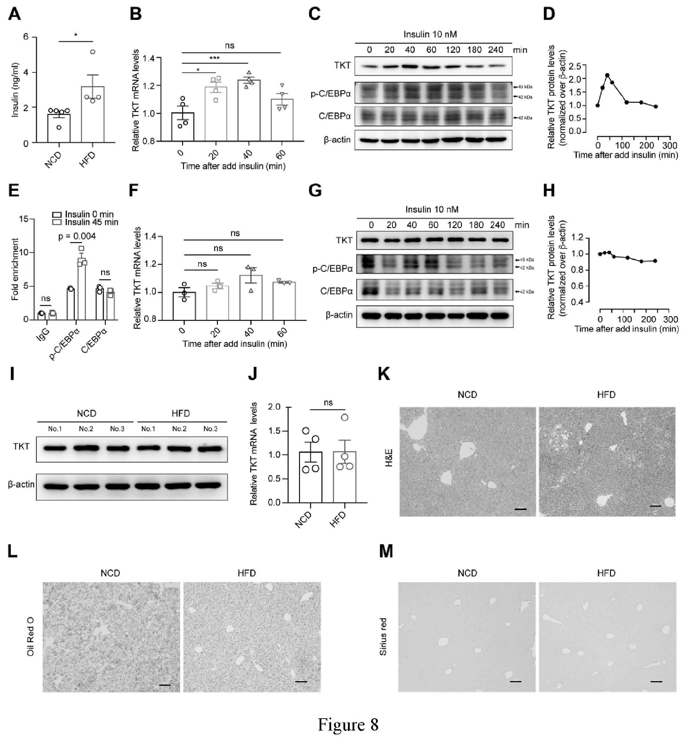

Figure 8. Hyperinsulinemia-induced elevation of hepatic TKT levels.
   (A) Serum insulin levels in wild-type mice after feeding with a normal chow diet (NCD) or a high-fat diet (HFD) for 90 days (NCD group, n = 5; HFD group, n = 4).
   (B-D) (B) TKT mRNA levels, (C) protein levels of TKT, p-C/EBPα, and C/EBPα, and (D) relative quantification of TKT protein of wild-type mouse primary hepatocytes treated with 10 nM insulin (n = 4).
   (E) Binding ability of p-C/EBPα and C/EBPα to the TKT promoter in wild-type mouse primary hepatocytes treated with 10 nM insulin (n = 3).
   (F-H) (F) TKT mRNA levels, (G) protein levels of TKT, p-C/EBPα, and C/EBPα, and (H) relative quantification of TKT protein of primary hepatocytes from liver-specific insulin receptor knockout mice treated with 10 nM insulin (n = 3).
   (I-M) Hepatic TKT (I) protein and (J) mRNA levels, (K) H&E staining, (L) Oil Red O staining, and (M) Sirius Red staining of liver tissue from liver-specific insulin receptor knockout mice after feeding with a normal chow diet or a high-fat diet for 90 days (n = 3 to 4).

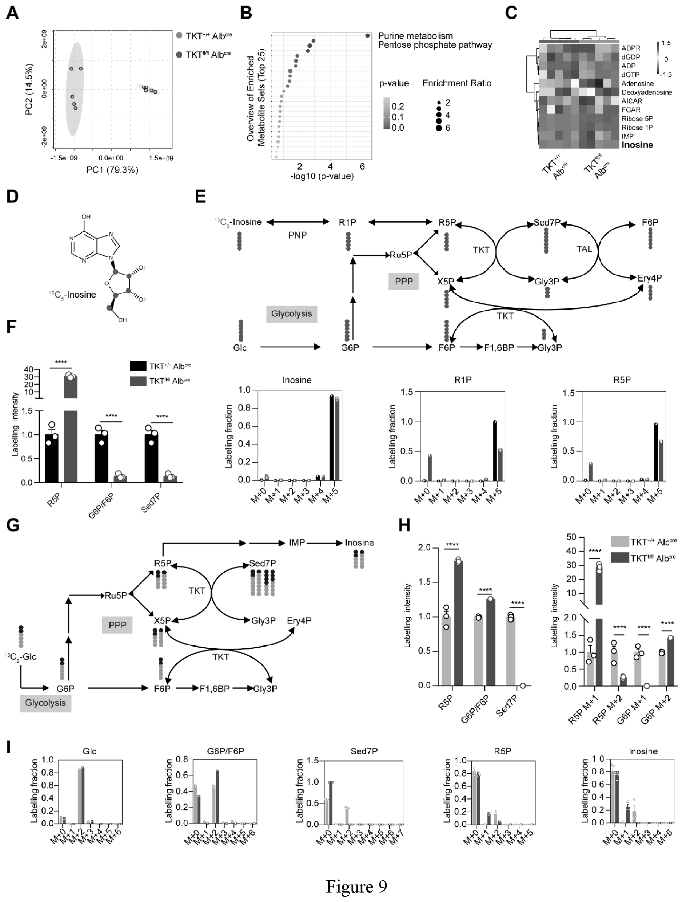

Figure 9. TKT-deficient hepatocytes show significant R5P and inosine accumulation.
   (A and B) (A) Principal component analysis (PCA) of metabolites and (B) KEGG metabolic pathway enrichment analysis in primary hepatocytes from wild-type mice and liver-specific TKT-deficient mice (n = 5).
   (C) Heatmap of significantly different metabolites in the purine metabolic pathway (n = 5).
   (D) Structural diagram of ¹³C-labeled inosine (¹³C₅-inosine).
   (E) Metabolic flux analysis of primary hepatocytes from wild-type and liver-specific TKT-deficient mice after culturing in medium containing 5.6 mM ¹³C₅-inosine for 6 hours (n = 3).
   (F) Isotopic labeling levels of R5P, G6P/F6P, and S7P in primary hepatocytes from wild-type and liver-specific TKT-deficient mice (n = 3).
   (G) Labeling patterns analysis of glucose and G6P/F6P in primary hepatocytes from wild-type and liver-specific TKT-deficient mice after culturing in medium containing 15 mM 1,2 ¹³C₂-glucose for 30 minutes, labeling patterns analysis of Sed7P, R5P, and R1P at 6 hours, and labeling patterns analysis of inosine at 48 hours (n = 3).
   (H and I) Labeling patterns of R5P, G6P/F6P, and S7P in primary hepatocytes from wild-type and liver-specific TKT-deficient mice (n = 3).

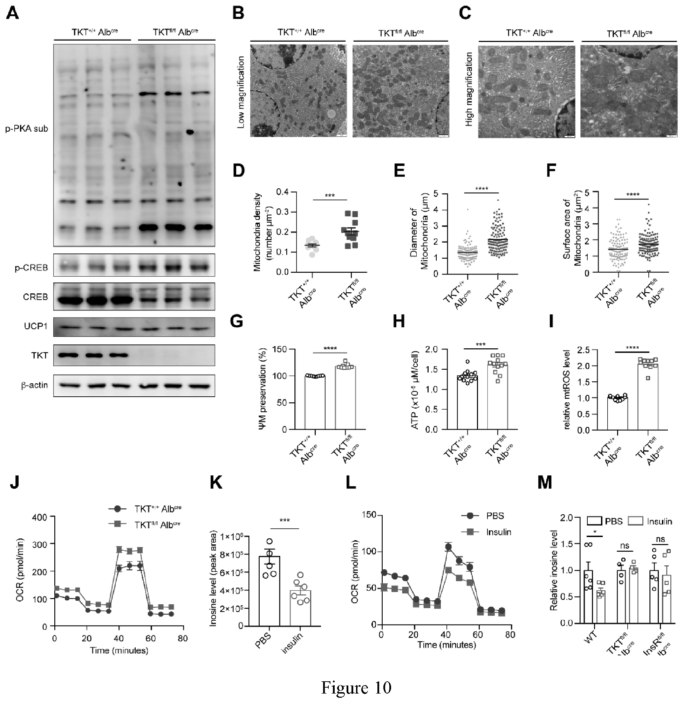

Figure 10. TKT deficiency enhances mitochondrial function by promoting the inosine-PKA-CREB pathway.
   (A) Expression levels of p-PKA substrate, p-CREB, CREB, UCP1, and TKT in primary hepatocytes from wild-type and liver-specific TKT-deficient mice (n = 3).
   (B-F) Analysis of mitochondrial morphology and number in liver tissues from wild-type and liver-specific TKT-deficient mice, including (B) low-magnification electron microscopy images, (C) high-magnification electron microscopy images, (D) mitochondrial density, (E) mitochondrial diameter, and (F) mitochondrial surface area (n = 3 for B and C, n = 10 for D, n = 120 for E and F).
   (G-J) Analysis of mitochondrial function in primary hepatocytes from wild-type and liver-specific TKT-deficient mice, including (G) mitochondrial membrane potential, (H) ATP, (I) mitochondrial ROS (mtROS), and (J) OCR levels (n = 10-12 for J, K, and L, n = 3 for M).
   (K and L) (K) Inosine and (L) OCR levels in primary hepatocytes from wild-type mice treated with 10 nM insulin (n = 6).
   (M) Inosine levels in primary hepatocytes from wild-type, liver-specific TKT-deficient, and liver-specific insulin receptor-deficient mice treated with 10 nM insulin (n = 4-6).

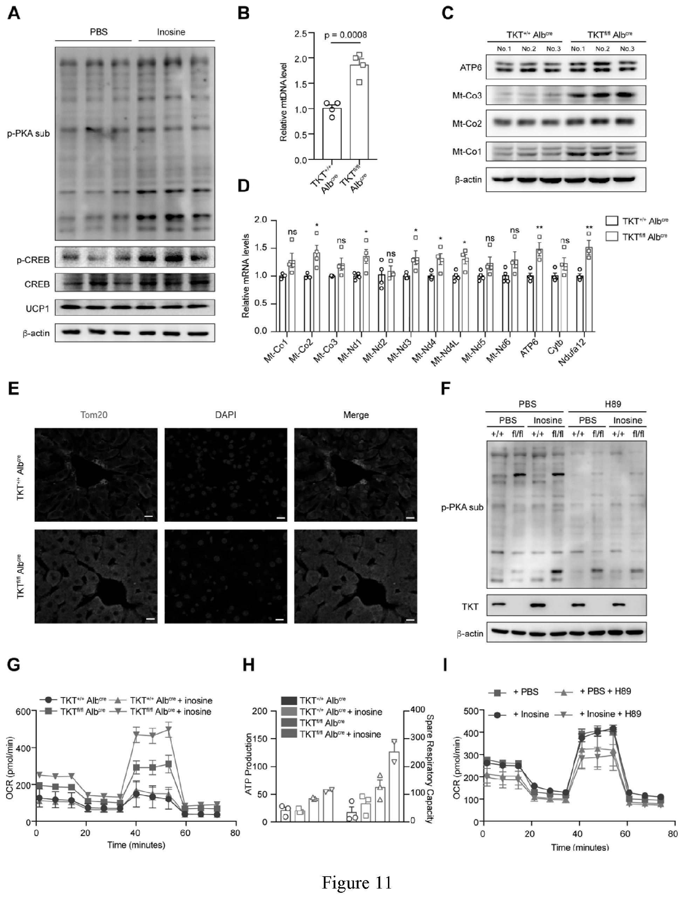

Figure 11. TKT deficiency enhances mitochondrial function by promoting the inosine-PKA-CREB pathway.
   (A) Expression levels of p-PKA substrate, p-CREB, CREB, and UCP1 proteins in primary hepatocytes from wild-type mice after treatment with 56 µM inosine for 6 hours (n = 3).
   (B-D) (B) Mitochondrial DNA (mtDNA) content, and protein (C) and mRNA (D) expression levels of mitochondrial genes in primary hepatocytes from wild-type and liver-specific TKT-deficient mice (n = 4 for B and D, n = 3 for C).
   (E) Immunofluorescence staining of the TOM20 protein in liver tissues from wild-type and liver-specific TKT-deficient mice (n = 3).
   (F) Western blot analysis of p-PKA substrate and TKT proteins in primary hepatocytes from wild-type mice after treatment with 56 µM inosine and 10 µM H89 for 6 hours.
   (G and H) Analysis of (G) OCR, (H) ATP, and spare respiratory capacity in primary hepatocytes from wild-type and liver-specific TKT-deficient mice after treatment with 56 µM inosine for 6 hours (n = 3).
   (I) OCR changes in primary hepatocytes from wild-type mice after treatment with 56 µM inosine and 10 µM H89 for 6 hours (n = 3).
   For all: *p < 0.05, **p < 0.01, ***p < 0.005, ****p < 0.001. Data are represented as mean ± SEM. Two-tailed t-test was applied for B and D.

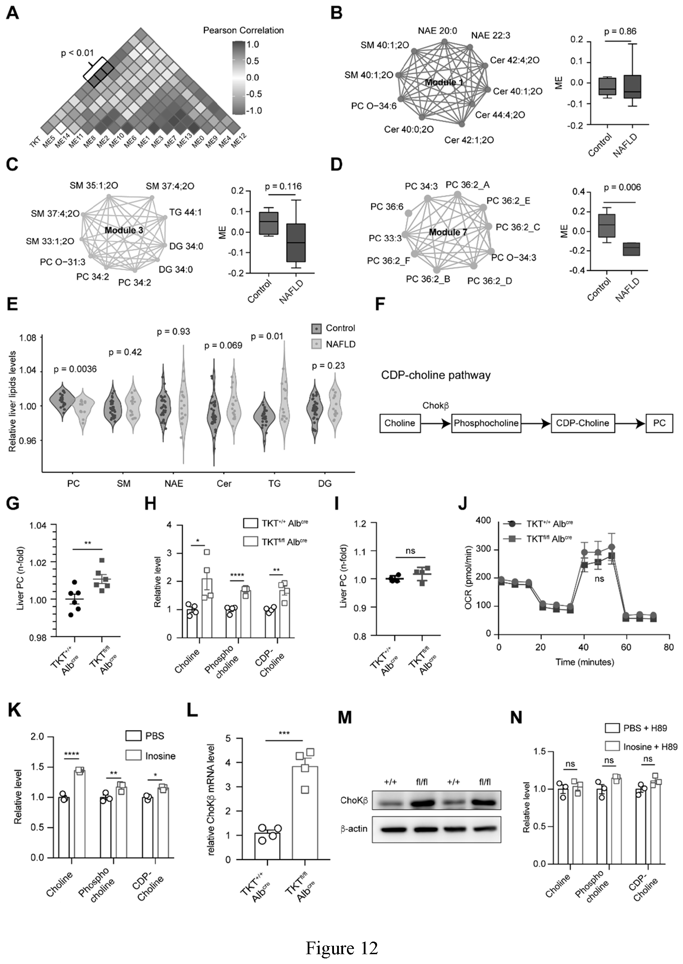

Figure 12. Inosine upregulates PC synthesis by activating the PKA-CREB-ChoKβ axis in hepatocytes.
   (A) Heatmap of the eigengene adjacency matrix. Each row or column corresponds to an eigengene, including module numbers and the TKT protein. Red and blue indicate positive and negative correlations, respectively. Modules 1, 3, and 7 show Pearson correlation coefficients > 0.72, with p < 0.01.
   (B-D) Network diagrams of the top 10 lipids with the highest connectivity in (B) Module 1, (C) Module 3, and (D) Module 7. Changes in module eigengenes (ME) in the livers of NAFLD patients and healthy controls in (B) Module 1, (C) Module 3, and (D) Module 7. The top and bottom lines of the box plots represent the maximum and minimum values based on Tukey's test, and the center line represents the median.
   (E) Levels of six lipids in Modules 1, 3, and 7 in the livers of NAFLD patients and healthy controls (n = 24).
   (F) Schematic diagram of the phosphatidylcholine synthesis pathway.
   (G and H) Levels of (G) phosphatidylcholine (PC) and (H) CDP-choline pathway metabolites in primary hepatocytes from wild-type and liver-specific TKT-deficient mice (n = 6 for G, n = 4 for H).
   (I and J) Levels of (I) PC and (J) OCR in primary hepatocytes from wild-type and liver-specific TKT-deficient mice after feeding with a low-choline diet (choline content at 20% of that in the NCD) for 3 weeks (n = 4 for I, n = 3 for J).
   (K) Relative levels of CDP-choline pathway metabolites in primary hepatocytes from wild-type mice after treatment with 56 µM inosine for 6 hours (n = 3).
   (L and M) (L) mRNA and (M) protein levels of ChoK in primary hepatocytes from wild-type and liver-specific TKT-deficient mice (L = 4, M = 2).
   (N) Relative levels of CDP-choline pathway metabolites in primary hepatocytes from wild-type mice after treatment with 56 µM inosine and 10 µM H89 for 6 hours (N = 3).
Figure 13. The target gene of the present disclosure and its upstream and downstream regulatory signaling pathways.
Figure 14. TKT deficiency enhances hepatocyte mitochondrial function by increasing phosphatidylcholine synthesis.
   (A) Protein levels of PGC1α, NRF2, and DRP1 in TKT-deficient hepatocytes.
   (B) Lipidomics results of mitochondria in TKT-deficient hepatocytes.
   (C-F) Levels of choline, phosphocholine, CDP-choline, and PC in liver tissues of mice in low-choline diet group or normal diet group.
   (G-J) Levels of mitochondrial membrane potential, ATP, mitochondrial ROS, and OCR in hepatocytes of mice in low-choline diet group or normal diet group.
   (K-P) Levels of (K) choline, (L) PC, and (M) CDP-choline in primary hepatocytes, and levels of (N) ATP, (O) mitochondrial membrane potential, and (P) mitochondrial ROS in hepatocytes from wild-type and liver-specific TKT-deficient mice fed with a low-choline diet.

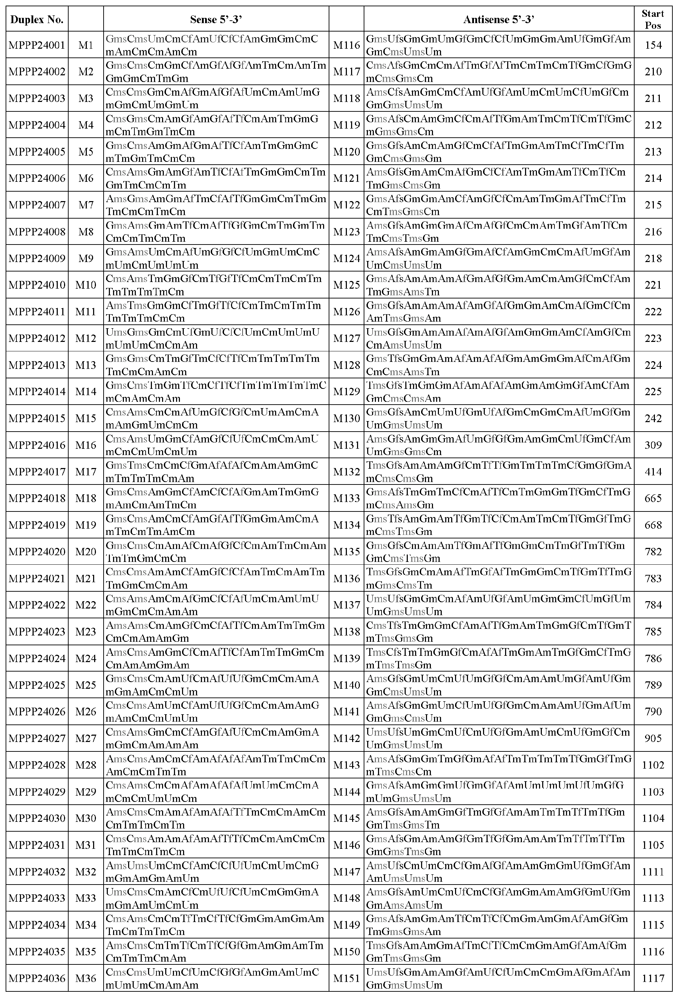

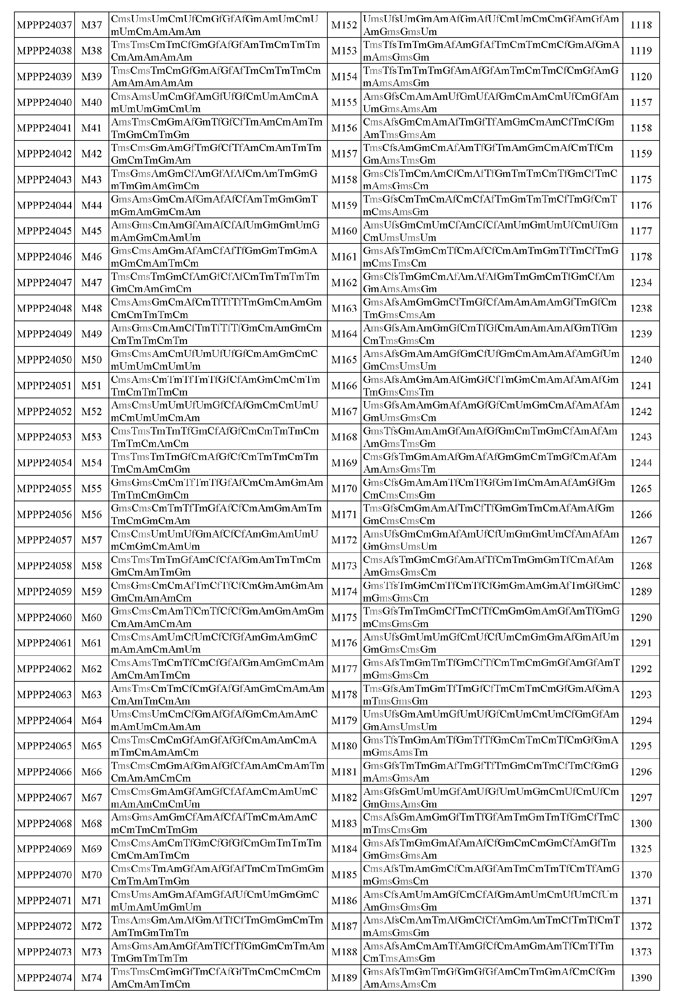

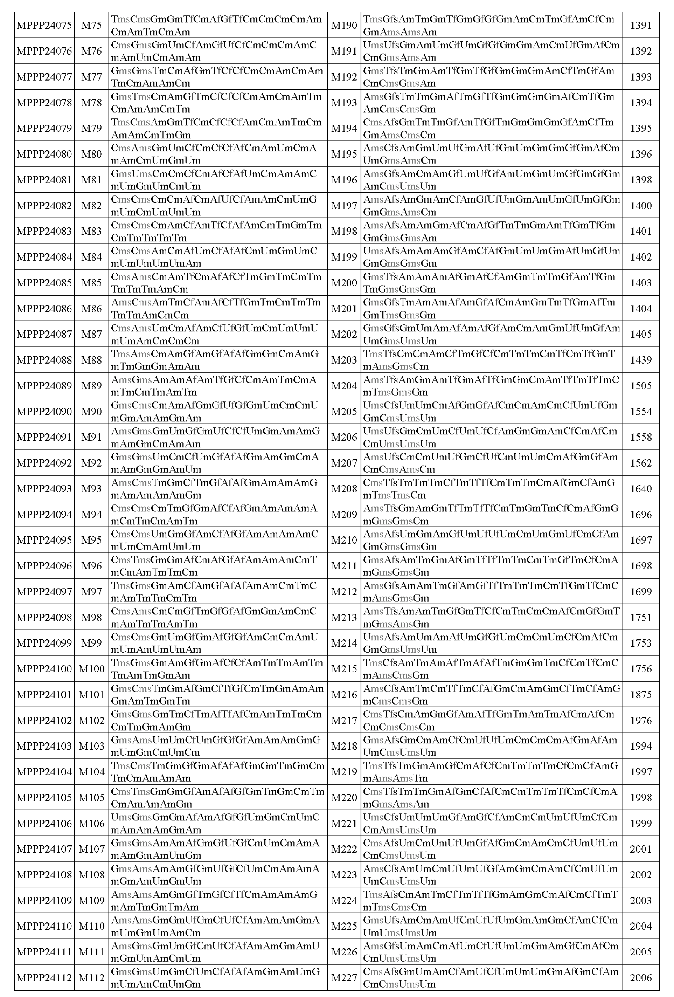

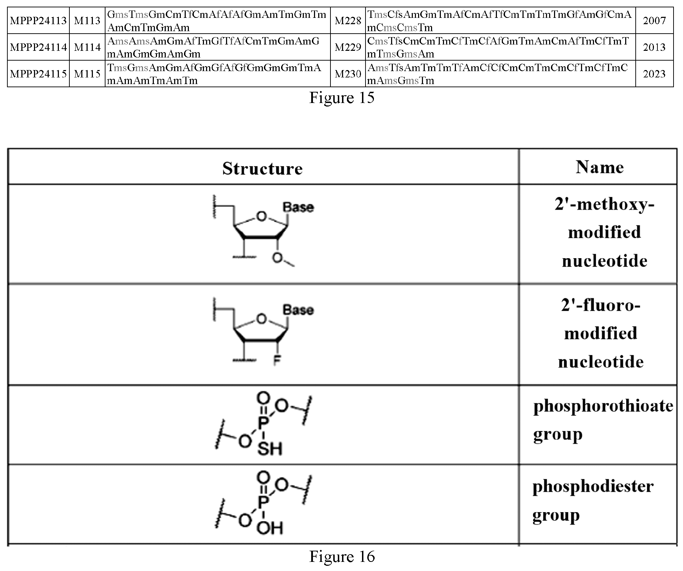

Figure 15. Modified sense and antisense strands prepared based on the sense strand of the siRNA from Table 2, with consideration of the corresponding antisense strand.
Figure 16. Modified groups; wherein Base denotes the nucleobase.

### DETAILED DESCRIPTION OF THE INVENTION

For the first time, the present invention discloses a novel target gene closely associated with the alleviation or treatment of metabolism-associated fatty liver disease and a signaling pathway associated with upstream and downstream regulation thereof. The present invention also discloses a new mechanism in which the signaling pathway is involved in regulating metabolism-associated fatty liver disease. The present invention further discloses a preferred medicament for the alleviation or treatment thereof.

### Transketolase (TKT) or the insulin/InsR-C/EBPα-TKT signaling pathway comprising TKT and the regulation thereof

As used herein, the terms "(signaling) pathway" and "(signaling) route" are used interchangeably.

As used herein, the term "(signaling) pathway" refers to a signaling system formed by mutual constraints or interactions among a series of genes, proteins or their metabolites (synthetic or processed products). It also comprises the interactions of pathway proteins with other intracellular components or organelles, and sometimes involves the co-participation of upstream or downstream genes or proteins thereof, which generally leads to the occurrence of certain cellular events. The insulin/InsR-C/EBPα-TKT signaling pathway mainly comprises the following essential elements: TKT gene/protein, C/EBPα gene/protein, and InsR gene/protein; preferably, it also comprises its upstream or downstream regulatory genes/proteins or chemical molecules.

The nucleotide sequence of the TKT gene is, for example, as shown in Gene ID: 7086 (human) or Gene ID: 21881 (mouse); its amino acid sequence is, for example, as shown in NCBI Reference Sequence: NP_001055.1 (human) or NCBI Reference Sequence: NP_033414.1 (mouse).

The nucleotide sequence of the C/EBPα gene is, for example, as shown in Gene ID: 1050 (human) or Gene ID: 12606 (mouse); its amino acid sequence is, for example, as shown in NCBI Reference Sequence: NP_004355.2 (human) or NCBI Reference Sequence: NP_031704.2 (mouse).

The nucleotide sequence of the InsR gene is, for example, as shown in Gene ID: 3643 (human) or Gene ID: 16337 (mouse); its amino acid sequence is, for example, as shown in NCBI Reference Sequence: NP_000199.2 (human) or NCBI Reference Sequence: NP_034698.2 (mouse).

In the present disclosure, unless otherwise specified, information on proteins/genes in certain upstream or downstream signaling pathways related to the insulin/InsR-C/EBPα-TKT signaling pathway is known in the art.

Within hepatocytes, downstream of the insulin/InsR-C/EBPα-TKT signaling pathway, there exist a series of further regulatory pathways. Downregulating the TKT or the insulin/InsR-C/EBPα-TKT signaling pathway comprising TKT can further: increase inosine levels and enhance mitochondrial function, thereby improving hepatic lipid metabolism, and alleviating or treating metabolism-associated fatty liver disease (MAFLD). More specifically, downregulating the TKT or the insulin/InsR-C/EBPα-TKT signaling pathway comprising TKT prevents R5P derived from inosine from entering glycolysis, promotes the synthesis of inosine from glucose-derived R5P, and elevates intracellular inosine levels, thereby promoting the inosine-PKA-CREB pathway, activating the PKA-CREB-ChoKβ axis, increasing phosphatidylcholine (PC) synthesis, enhancing mitochondrial function, improving hepatic lipid metabolism, and alleviating or treating MAFLD; preferably, the increasing phosphatidylcholine (PC) synthesis is mediated by the CDP-choline pathway.

As used herein, unless otherwise specified, the discussed target gene/protein and the signaling pathway in which it is involved are those in the liver (hepatocytes).

When used as a target for artificial regulation or for establishing an artificial screening system, the above proteins or the genes encoding them may be naturally occurring, for example, they can be purified and isolated from mammals; or they may be recombinantly prepared, for example, recombinant proteins can be produced according to conventional genetic recombination techniques. In addition, any variant forms that do not affect the biological activity of these proteins are usable, such as derivatives or variants whose function has not been altered.

The above proteins (polypeptides) also include variant forms thereof, including (but not limited to): deletions, insertions, and/or substitutions of several (typically 1-50, preferably 1-30, more preferably 1-20, most preferably 1-10, and even more preferably such as 1-8, 1-5) amino acids, as well as addition or deletion of one or several (typically within 20, preferably within 10, more preferably within 5) amino acids at the C-terminus and/or N-terminus. Any protein that has high homology to said protein (for example, 70% or higher homology with the polypeptide sequence; preferably 80% or higher homology; more preferably 90% or higher homology, such as 95%, 98%, or 99% homology) and possesses the same function as said protein is also included in the present disclosure. The present disclosure also includes a mutant form of the protein or a truncated form of the protein, provided that the mutant or truncated form substantially retains the function of the full-length protein.

The sequences of the above genes also include degenerate sequences thereof. The polynucleotides (genes) encoding the proteins may be natural genes or degenerate sequences thereof.

As used herein, the terms "downregulation (downregulator)" and " inhibition (inhibitor)" are used interchangeably, and also include " blocking (blocker)", "antagonism (antagonist)", and the like.

The present inventors have found that, within the signaling pathway, downregulating TKT, downregulating the C/EBPα-TKT interaction, or downregulating the insulin/InsR-C/EBPα interaction can alleviate or treat metabolism-associated fatty liver disease. Therefore, this mode of action can be used to screen or design drugs suitable for targeted regulation.

It should be understood that, after learning the function of the insulin/InsR-C/EBPα-TKT signaling pathway (preferably, also including its upstream or downstream proteins or genes), various methods well known to those skilled in the art can be employed to modulate the insulin/InsR-C/EBPα-TKT signaling pathway. For example, various methods well known to those skilled in the art can be used to modulate the expression of pathway proteins or to achieve deletion of their expression.

The present disclosure provides a method for downregulating the insulin/InsR-C/EBPα-TKT signaling pathway, comprising targeted mutation, gene editing, or genetic recombination of the TKT, C/EBPα, or InsR gene in the insulin/InsR-C/EBPα-TKT signaling pathway, thereby achieving downregulation.

As a more specific embodiment, a method for downregulating the expression of the TKT, C/EBPα, or InsR gene is provided, comprising: introducing into cells an interfering molecule that interferes with the expression of the TKT, C/EBPα, or InsR gene, or treating the cells by an appropriate route to allow the interfering molecule to be introduced into the cells, for example, designing a cell-penetrating domain to confer cell-penetrating ability on the interfering molecule. RNA interference technology is a technique for silencing gene expression. The principle of RNA interference technology is that long double-stranded RNA is cleaved by the specific nuclease Dicer into small interfering RNAs of 21 to 23 nt consisting of sense and antisense strands. The small interfering RNA then forms an RNA-induced silencing complex (RISC) and is unwound into single strands. The antisense strand guides the silencing complex to specifically bind to the target mRNA through base pairing, resulting in degradation of the mRNA.

As another embodiment of the present disclosure, a CRISPR/Cas system is used for gene editing to knock down, knock out, or downregulate the target gene. Appropriate sgRNA target sites lead to higher gene editing efficiency, so before proceeding with gene editing, suitable target sites need to be designed and identified. After designing specific target sites, in vitro cellular activity screening is also required to obtain effective target sites for subsequent experiments. Using this method, the TKT, C/EBPα, or InsR gene can be converted into a truncated or mutant form with loss of function.

### Regulatory agent and pharmaceutical composition

As a preferred embodiment of the present disclosure, a downregulator that downregulates the expression or activity of TKT, downregulates the C/EBPα-TKT interaction, or downregulates the insulin/InsR-C/EBPα interaction is provided. The downregulator refers to any substance that reduces the activity, reduces the stability, downregulates the expression, reduces the activation level (e.g., reduces the phosphorylation level), shortens the effective action time, or inhibits the transcription or translation of TKT, C/EBPα, or InsR. These substances can all be used in the present disclosure as the substances potentially useful for alleviating or treating metabolism-associated fatty liver disease. They can be compounds, small chemical molecules, or biomolecules. The biomolecules can be at the nucleic acid level (including DNA, RNA) or at the protein level.

As used herein, the term "metabolism-associated fatty liver disease" includes fatty liver.

As used herein, the term "metabolism-associated fatty liver disease" includes two different indications: non-alcoholic fatty liver disease and non-alcoholic steatohepatitis.

In the present disclosure, the downregulator can be: a nucleic acid inhibitor, a protein inhibitor, an antibody, a ligand, a compound, a nuclease, a nucleic acid binding molecule, etc., provided that it can downregulate the expression of TKT, C/EBPα, or InsR, inhibit its activity or function, or downregulate the C/EBPα-TKT interaction or the insulin/InsR-C/EBPα interaction. The nucleic acid inhibitor includes: an shRNA, an antisense nucleic acid, a small interfering RNA, a microRNA that targets the coding gene or transcript of TKT, C/EBPα, or InsR for inhibition or silencing, or a construct capable of expressing or forming the shRNA, antisense nucleic acid, small interfering RNA, or microRNA.

For example, the downregulator is: an interfering RNA molecule or an antisense nucleotide that specifically interferes with the expression of TKT, C/EBPα, or InsR or its upstream genes; or a homologous recombination, targeted mutation, or gene editing reagent that specifically targets TKT, C/EBPα, or InsR or its upstream genes, and the like.

As a preferred embodiment of the present disclosure, the downregulator is an interfering molecule that specifically interferes with TKT expression. Short hairpin RNA (shRNA) is an RNA sequence that forms a tight hairpin structure and can silence gene expression via RNA interference. The interfering molecule that specifically interferes with the expression of the TKT, C/EBPα, or InsR gene can be an shRNA molecule targeting the TKT, C/EBPα, or InsR gene, or an siRNA molecule targeting the TKT, C/EBPα, or InsR gene, and the like.

Focusing on TKT as a target, the present inventors have conducted in-depth experimental validation, including validation at both the cellular level and the animal level. Based on this, the present inventors have optimized and obtained a series of siRNA reagents with excellent efficacy. The siRNA is an siRNA having a nucleotide sequence as shown in any one of SEQ ID NO: 1 to SEQ ID NO: 50, and SEQ ID NO: 53 to SEQ ID NO: 167; more preferably, the siRNA is an siRNA having a nucleotide sequence as shown in any one of SEQ ID NO: 37 (No. 1115), SEQ ID NO: 25 (No. 773), SEQ ID NO: 50 (siTKT16), SEQ ID NO: 49, SEQ ID NO: 61, SEQ ID NO: 63, SEQ ID NO: 73, SEQ ID NO: 74, SEQ ID NO: 78, SEQ ID NO: 88, or SEQ ID NO: 130 (siTKT10). The siRNA can be used in multiple species and has highly desirable targeted regulatory effects without non-specific regulation, and possesses high clinical application value.

Considering that the disease addressed by the present disclosure is a liver disease, in a preferred embodiment, the siRNA is formulated as a stable formulation for hepatocyte-targeted delivery.

In a particularly preferred embodiment, the siRNA, after fluoro and methoxy modification, is covalently conjugated to N-acetylgalactosamine (GalNAc) to form a conjugate.

As another alternative embodiment of the present disclosure, the downregulator is an inhibitor of mutation, gene editing, or genetic recombination that targets TKT, C/EBPα, or InsR. As a more specific embodiment, by any of the above methods, TKT, C/EBPα, or InsR is converted into a mutant form thereof, thereby rendering it no longer functional.

The present disclosure also provides a pharmaceutical composition for alleviating or treating metabolism-associated fatty liver disease, comprising an effective amount of the downregulator according to the present disclosure.

As used herein, the term "effective amount" or "effective dose" refers to an amount that produces a function or activity in humans and/or animals and is acceptable to humans and/or animals, as used herein.

As used herein, a "pharmaceutically acceptable" ingredient is one that is suitable for use in humans and/or mammals without undue adverse side effects (such as toxicity, irritation, and allergic reactions), i.e., a substance with a reasonable benefit/risk ratio. The term "pharmaceutically acceptable carrier" refers to a carrier used for the administration of therapeutic agents, including various excipients and diluents.

The present disclosure also provides a kit for alleviating or treating metabolism-associated fatty liver disease, the kit comprising: an effective amount of the downregulator according to the present disclosure. More preferably, the kit further comprises: instructions for use to guide clinicians in administering the drug in a correct and appropriate manner.

For ease of administration, the downregulator is prepared in the form of a unit dosage and placed in a kit. "Unit dosage" refers to a dosage form prepared as a single administration for convenience of medication, including but not limited to various liquid dosage forms (e.g., injections), solid dosage forms (e.g., tablets), capsules, and sustained-release formulations. In addition, the downregulator may also be independently placed in separate containers and mixed when needed for application.

Although specific examples of the present disclosure provide dosing regimens for animals such as mice, it should be understood that converting the dosage administered to an animal such as a mouse to a dosage suitable for human administration is readily achievable by those skilled in the art, for example, by calculation according to the Meeh-Rubner formula: A = k×(W^{2/3})/10,000, where A is body surface area in m², W is body weight in grams, and k is a constant that varies depending on the animal species, including but not limited to: 9.1 for mice and rats, 9.8 for guinea pigs, 10.1 for rabbits, 9.9 for cats, 11.2 for dogs, 11.8 for monkeys, and 10.6 for humans. It should be understood that the conversion of dosage may vary depending on the drug and clinical circumstances, as assessed by an experienced pharmacist.

### Drug screening based on the InsR-C/EBPα-TKT signaling pathway

Based on the novel discoveries of the present inventors, research on the (insulin/)InsR-C/EBPα-TKT signaling pathway has various uses, including: screening for a substance that regulates this signaling pathway, with the aim of alleviating or treating metabolism-associated fatty liver disease. Wherein, the modulation includes: downregulating TKT, downregulating the C/EBPα-TKT interaction, downregulating the insulin/InsR-C/EBPα interaction, and the like.

The present disclosure provides a method for screening a downregulator for regulating the InsR-C/EBPα-TKT signaling pathway, by adding a candidate substance to be screened to a system comprising the InsR-C/EBPα-TKT signaling pathway, and observing changes or interactions of proteins or genes in the InsR-C/EBPα-TKT signaling pathway. If the candidate substance has the effect of downregulating TKT, downregulating the C/EBPα-TKT interaction, downregulating the insulin/InsR-C/EBPα interaction, or the like, then the candidate substance is a substance useful for alleviating or treating metabolism-associated fatty liver disease.

As used herein, terms such as "inhibiting" and "downregulating" refer to "inhibiting" and "downregulating" that are statistically significant, i.e., a significant "inhibition" or "downregulation". For example, compared with the protein activity, protein expression, protein binding, or methylation level of a control group, the "inhibition" or "downregulation" is a significant reduction of more than 10%, 20%, 30%, 40%, or 50%; more preferably of more than 60%, 70%, or 80%.

The system comprising the InsR-C/EBPα-TKT signaling pathway is selected from: a cellular system (or cell culture system), a subcellular system (or subcellular culture system), a solution system, an animal system, or a tissue system (or tissue culture system). Preferably, the system containing the InsR-C/EBPα-TKT signaling pathway is a hepatocyte (or cell culture thereof).

As a preferred embodiment of the present disclosure, the method further comprises: performing further cellular experiments and/or animal experiments on the obtained potential substances, so as to further select and identify substances that are useful for alleviating or treating metabolism-associated fatty liver disease from the candidate substances.

When performing screening, various techniques well known in the art can be employed to determine changes and interactions of the protein or its encoding gene.

A variety of conventional techniques can be used to assess the transcription or expression of genes in the system. These techniques include, but are not limited to: oligonucleotide hybridization techniques (e.g., probes), polymerase chain reaction (PCR), polyacrylamide gel electrophoresis, and the like. For detecting protein-protein interactions and the strength of such interactions, various techniques well known to those skilled in the art can be employed, such as co-immunoprecipitation, GST pull-down assays, phage display technology, or the yeast two-hybrid system. Protein nuclear localization is also a technique well known in the art.

Substances preliminarily screened out by the above methods can constitute a screening library, so that people can ultimately select therefrom substances that are truly useful for alleviating or treating metabolism-associated fatty liver disease.

The present disclosure also provides a potential substance obtainable by the screening method described above for alleviating or treating metabolism-associated fatty liver disease.

The present disclosure also provides a method for preparing a medicament for alleviating or treating metabolism-associated fatty liver disease (particularly for alleviating or treating metabolism-associated fatty liver disease), the method comprising: synthesizing and/or purifying a substance useful for alleviating or treating metabolism-associated fatty liver disease obtained by the screening described above, as a medicament for alleviating or treating metabolism-associated fatty liver disease.

The obtained substance useful for alleviating or treating metabolism-associated fatty liver disease can be used for preparing a pharmaceutical composition, as described in the following text of the present disclosure.

When a protein, a gene, or a specific region thereof is used as a target, methods for screening a substance that acts on the target are well known to those skilled in the art, and these methods can all be used in the present disclosure. The candidate substance can be selected from: peptides, polypeptides, peptidomimetics, non-peptide compounds, carbohydrates, lipids, antibodies or antibody fragments, ligands, small organic molecules, small inorganic molecules, nucleic acid sequences, and the like. Depending on the type of substance to be screened, those skilled in the art are aware of how to select an appropriate screening method.

### Application in diagnosis or prognosis assessment

The present disclosure has found that upregulation of hepatic TKT is a common feature of NAFLD in both humans and mice, that upregulation of TKT promotes the progression of NAFLD in mice, that upregulation of TKT promotes non-alcoholic steatohepatitis (NASH), and that TKT is involved in a novel signaling pathway: the insulin/InsR-C/EBPα-TKT signaling pathway. Therefore, TKT and the signaling pathway in which it is involved can serve as molecular markers for guiding the diagnosis or prognosis (including medication guidance) of metabolism-associated fatty liver disease (MAFLD): (i) for disease subtyping and differential diagnosis; (ii) for evaluating therapeutic drugs, drug efficacy, and prognosis in relevant populations, as well as for selecting appropriate treatment methods. For example, populations with abnormal (elevated) TKT gene expression can be identified, thereby enabling more targeted treatment.

By determining the expression level or activity level of TKT or pathway genes/proteins of the signaling pathway in which TKT is involved in the test sample, the disease prognosis of a subject from which a test sample is derived can be predicted, and an appropriate drug can be selected for treatment. Generally, a threshold for TKT expression can be established. When the expression level of TKT is higher than the established threshold, a treatment regimen involving inhibition of TKT is considered. The threshold is readily determinable by those skilled in the art; for example, a threshold for abnormal TKT expression can be obtained by comparing and analyzing the general TKT expression level in patients with metabolism-associated fatty liver disease or that in normal healthy individuals.

Therefore, the present disclosure provides use of TKT or a pathway gene/protein of the signaling pathway in which TKT is involved, in preparing a reagent or a kit for prognosis assessment of metabolism-associated fatty liver disease. Various techniques known in the art can be employed to detect the presence or absence and expression level of the corresponding gene or protein, and these techniques are all encompassed by the present disclosure. For example, existing techniques such as Southern blotting, Western blotting, DNA sequence analysis, PCR, and the like can be used, and these methods can be used in combination. The present disclosure also provides reagents for detecting the presence or absence and expression level of the gene or protein in an analyte. Preferably, when performing detection at the gene level, specifically amplifying primers or specifically recognizing probes can be used to determine the presence or absence of the target gene; when performing detection at the protein level, antibodies or ligands that specifically bind to the protein can be used to determine the expression level of the target protein.

The kit may also include various reagents required for DNA extraction, PCR, hybridization, color development, etc., including but not limited to: extraction solutions, amplification solutions, hybridization solutions, enzymes, control solutions, color development solutions, washing solutions, and the like. In addition, the kit may also include instructions for use and/or nucleic acid sequence analysis software, and the like.

The present disclosure is further illustrated below in conjunction with specific embodiments. It should be understood that these embodiments are only intended to illustrate the present disclosure and are not intended to limit the scope thereof. In the following examples, experimental methods without specified conditions are generally performed under conventional conditions, such as those described in J. Sambrook et al., Molecular Cloning: A Laboratory Manual, 3rd edition, Science Press, or under conditions recommended by the manufacturer.

### Example 1. Expression characteristics of TKT in liver tissue

Liver samples were obtained from patients with non-alcoholic fatty liver disease (NAFLD) or healthy liver transplant donors, with sample source information shown in Figure 2A. The samples were divided into a NAFLD group (HS) and a healthy control group (Control) based on clinicopathological diagnosis.

Metabolomics analysis showed that, compared with the control group, the pentose phosphate pathway (PPP) was significantly altered in NAFLD livers (Figure 1A).

According to further proteomic analysis of PPP and glycolytic enzymes, the non-oxidative PPP metabolic enzyme TKT exhibited the most significant difference between NAFLD livers and the control group (Figure 1B).

For the expression of the non-oxidative PPP metabolic enzyme TKT, Western blot and quantitative PCR analyses were performed. The results showed that TKT was significantly upregulated in NAFLD livers (Figures 1C-E).

Immunohistochemistry (IHC) analysis was performed on a set of randomly selected human liver samples (n = 6), and the results showed that TKT staining was stronger in the NAFLD group than in the healthy control group (Figure 1F).

Furthermore, NAFLD was induced in wild-type mice by feeding them with a high-fat diet (HFD). Mice in the HFD group were fed with a high-fat diet daily with free access to food; mice in the control NCD group were fed with a normal diet daily with free access to food. This simulated the onset and progression of NAFLD in human patients. After 3 months, lipid accumulation and TKT upregulation were observed in the mouse livers (Figures 1G-J).

Therefore, these findings indicate that upregulation of hepatic TKT is a shared characteristic of NAFLD in both humans and mice.

### Example 2. Upregulation of TKT promotes the progression of NAFLD in mice

### 1. Upregulation of hepatic TKT promotes non-alcoholic fatty liver (NAFL)

To investigate whether TKT upregulation drives the progression of NAFLD, an adenovirus (adeno-associated virus, AAV)-mediated gene overexpression system was constructed to overexpress TKT in mouse livers.

Tail vein injection of AAV-Flag-TKT into mice resulted in a significant increase in hepatic TKT levels (Figures 1K and 2B).

After 10 weeks of HFD, mice injected with AAV-Flag-TKT exhibited more severe hepatic steatosis (Figures 1L and 2C), as well as higher liver and serum triglyceride levels (Figure 1M).

Hepatic TKT overexpression also increased the levels of blood total cholesterol, serum alanine aminotransferase (ALT), and aspartate aminotransferase (AST) in mice (Figure 2D).

These results indicate that upregulation of hepatic TKT promotes high-fat diet-induced non-alcoholic fatty liver (NAFL).

### 2. Upregulation of hepatic TKT promotes non-alcoholic steatohepatitis (NASH)

The methionine- and choline-deficient diet (MCD) is commonly used to induce NASH in mice, which is a progressive form of NAFLD. Mice were fed with an MCD diet daily (with free access to food), and various parameters were measured in animals injected with AAV-Flag-TKT or the control (AAV-ctrl).

The results showed that after 4 weeks of MCD, mice injected with AAV-Flag-TKT exhibited more severe hepatocyte ballooning degeneration, lipid accumulation, inflammation, and fibrosis compared with the control group (Figure 2E), accompanied by elevated levels of liver triglycerides (TG) and total cholesterol (TC), as well as serum aspartate aminotransferase (AST) levels (Figure 2F), indicating that animals injected with AAV-Flag-TKT developed more significant NASH progression than the controls.

Therefore, upregulation of hepatic TKT significantly drives the progression of both NAFL and NASH.

### Example 3. Hepatocyte-specific TKT knockout ameliorates NAFLD in mice

### 1. Downregulation of hepatic TKT ameliorates non-alcoholic fatty liver (NAFL)

To investigate whether TKT is essential for the development of NAFLD, a hepatocyte-specific TKT knockout mouse strain (TKT^{flox/flox} Alb^{cre}, abbreviated as TKT^{fl/fl} Alb^{cre}) was generated.

Generation of TKT^{flox/flox} Alb^{cre} mice: The mouse strain was C57BL/6. LoxP sites were inserted between exons 1 and 2 and between exons 2 and 3 of the mouse TKT gene to obtain the TKT^{flox/flox} mouse strain (Figure 3S). This strain was then crossed with Alb^{cre} mice to generate TKT^{flox/flox} Alb^{cre} mice.

Generation of TKT^{+/+} Alb^{cre} mice: TKT^{+/+} Alb^{cre} mice were obtained by crossing wild-type C57BL/6 mice with Alb^{cre} mice.

Under normal chow diet (NCD) conditions, TKT^{flox/flox} Alb^{cre} mice exhibited normal liver morphology and function. TKT^{flox/flox} Alb^{cre} and TKT^{+/+} Alb^{cre} mice were fed with a high-fat diet (HFD) for 90 days. Compared with obese TKT^{+/+} Alb^{cre} mice, TKT^{flox/flox} Alb^{cre} mice showed a slower rate of body weight gain (Figure 4A). After 90 days of HFD feeding, TKT^{flox/flox} Alb^{cre} mice had lower body weight and liver weight compared with control mice (Figures 4B-D).

HFD-fed TKT^{flox/flox} Alb^{cre} mice showed milder hepatic steatosis compared with control mice, as confirmed by magnetic resonance imaging (MRI), liver appearance photographs, H&E and Oil Red O staining (Figures 3A-C).

Furthermore, liver triglyceride (TG) levels, serum triglycerides (TG), total cholesterol (TC), alanine aminotransferase (ALT), and aspartate aminotransferase (AST) in TKT^{flox/flox} Alb^{cre} mice were all lower than those in the control group (Figures 3D-H), while liver total cholesterol remained unchanged (Figure 3I).

Therefore, hepatocyte-specific TKT deficiency in mice significantly ameliorates HFD-induced NAFL.

### 2. Downregulation of hepatic TKT alleviates non-alcoholic steatohepatitis (NASH)

To investigate whether hepatocyte-specific TKT knockout can alleviate MCD diet-induced NASH, mice were fed with an MCD, and disease progression was observed in TKT-deficient mice (TKT^{flox/flox} Alb^{cre}) compared with control mice (TKT^{+/+} Alb^{cre}).

After 4 weeks of MCD feeding, TKT-deficient mice (TKT^{flox/flox} Alb^{cre}) showed significantly reduced hepatic lipid accumulation (Figures 3J, K), while the mRNA levels of lipid synthesis enzyme genes were unchanged (Figure 3L).

TKT^{flox/flox} Alb^{cre} mice showed far less inflammatory cell infiltration in the liver compared with control mice (Figures 3M, N), accompanied by downregulation of inflammation-related genes (Figure 3O).

MCD feeding resulted in severe liver fibrosis in control mice, as confirmed by Sirius Red staining and Masson staining, whereas TKT knockout in the liver alleviated this condition (Figure 3P and Figure 4E).

Furthermore, TKT deficiency also reduced hepatocyte necrosis associated with NASH progression (Figure 4F).

Therefore, TKT deficiency in hepatocytes ameliorates both NAFL and NASH in mice.

### 3. The role of hepatic TKT in the development of NAFLD

To further demonstrate that TKT is a driver of NAFLD, TKT was re-expressed in the livers of TKT^{flox/flox} Alb^{cre} mice using an adenovirus overexpression system. HFD-induced NAFL and MCD-induced NASH models were established in hepatocyte-specific TKT-deficient mice (TKT^{flox/flox} Alb^{cre}), followed by tail vein injection of adeno-associated virus AAV-Flag-TKT or AAV-Ctrl, respectively.

During HFD feeding, re-expression of hepatic TKT promoted hepatic lipid accumulation (Figures 4G, H), elevated serum and liver triglyceride and total cholesterol levels, as well as serum ALT and AST levels (Figures 4I-N).

Consistently, re-expression of hepatic TKT restored MCD-induced hepatic lipid accumulation and fibrosis, and upregulated liver triglyceride and cholesterol levels, as well as serum ALT and AST levels (Figures 4P-U).

In summary, upregulation of hepatic TKT is both necessary and sufficient for the development of NAFLD in mice.

### Example 4. Analysis of the regulatory role of TKT in the liver and the signaling pathways in which it is involved

### 1. Insulin-InsR signaling drives TKT upregulation and lipid accumulation during NAFLD progression

The inventors investigated the mechanism underlying TKT upregulation in NAFLD livers, as NAFLD was commonly associated with hyperinsulinemia and insulin resistance. Mice fed with a HFD exhibited elevated serum insulin levels (Figure 8A). In insulin-treated primary hepatocytes, TKT mRNA and protein levels peaked at 40 minutes and then gradually decreased (Figures 8B-D), accompanied by fluctuations in the levels of phosphorylated transcription factor CCAAT/enhancer-binding protein α (C/EBPα) (Figure 8C). Analysis suggested that C/EBPα was a candidate transcription factor for the TKT gene promoter.

Chromatin immunoprecipitation (ChIP) confirmed that insulin, through InsR, enhanced the binding ability of phosphorylated C/EBPα to the upstream 643-795 bp region of the TKT promoter (Figure 8E).

To further investigate the role of the insulin receptor (InsR) in insulin-induced TKT expression, a mouse strain (InsR^{flox/flox} Alb^{cre}) was generated in which InsR was specifically deleted in hepatocytes.

Generation of InsR^{flox/flox} Alb^{cre} mice: purchased from The Jackson Laboratory.

InsR^{flox/flox} Alb^{cre} primary hepatocytes did not show insulin-stimulated fluctuations in TKT and phosphorylated C/EBPα levels (Figures 8F-H), suggesting that insulin promoted TKT expression through InsR. Subsequently, InsR^{flox/flox} Alb^{cre} mice were fed with a HFD, and it was found that HFD neither increased hepatic TKT mRNA and protein levels (Figures 8I, J) nor induced significant lipid accumulation and fibrosis in the livers of InsR^{-/-} mice (Figures 2K-M).

Therefore, during NAFLD progression, insulin-InsR signaling drives TKT upregulation and lipid accumulation.

### 2. TKT-deficient hepatocytes feature R5P and inosine accumulation

Primary hepatocytes isolated from TKT^{flox/flox} Alb^{cre} and TKT^{+/+} Alb^{cre} mice were subjected to metabolomics analysis, and the PCA results showed significant differences between the two groups (Figure 9A).

Metabolic pathway enrichment analysis revealed that TKT deficiency significantly altered the purine metabolism pathway, as well as the PPP (Figure 9B). TKT deficiency upregulated the levels of nucleosides such as inosine, adenosine, and deoxyadenosine, particularly inosine (Figure 9C). It has been reported that inosine not only provides ribose as an alternative carbon source for central carbon metabolism through the PPP, but also acts as an extracellular signal to promote mitochondrial function in adipose tissue through the cAMP-PKA-UCP1 pathway.

Next, how TKT deficiency regulates hepatic inosine metabolism was investigated. Primary hepatocytes from TKT^{flox/flox} Alb^{cre} and TKT^{+/+} Alb^{cre} mice were cultured with [¹³C₅] inosine, in which only the ribose moiety contained ¹³C, for metabolic flux analysis.

The results confirmed that [¹³C₅] labeled inosine could be taken up by primary hepatocytes without interference from TKT deficiency (Figure 9D, inosine). TKT deficiency prevented carbon transfer among R5P, S7P, and F6P, resulting in an increased relative abundance of inosine-derived R5P (Figure 9F), while the relative abundances of F6P and S7P were decreased (Figure 9F). The elevated R5P level may inhibit the decomposition rate of the PNP enzyme, leading to reduced isotopic labeling efficiency of R1P and R5P (Figure 9E). These results indicate that TKT deficiency in hepatocytes impairs the entry of inosine-derived pentose into central carbon metabolism by blocking the non-oxidative PPP.

In hepatocytes, glucose is metabolized through the PPP to generate R5P for de novo IMP synthesis, and IMP is dephosphorylated to produce inosine. Next, how TKT deficiency affects the synthesis of glucose-derived inosine was investigated. TKT^{flox/flox} Alb^{cre} and TKT^{+/+} Alb^{cre} primary hepatocytes were treated with [1,2-¹³C₂] glucose for glucose metabolic flux analysis. Considering the two reactions catalyzed by TKT, TKT deficiency not only hindered carbon transfer between F6P and X5P, resulting in increased relative abundance and isotopic labeling efficiency of F6P (Figures 9G and 9H), but also decreased the relative abundance and isotopic labeling efficiency of S7P (Figure 9I). Furthermore, the major isotopic labeling form of R5P shifted from M+2 to M+1 (Figure 9I), suggesting that the compensatory oxidative PPP may produce R5P with higher abundance (Figure 9H). Collectively, the accumulation of R5P in TKT-deficient hepatocytes provides more pentose for inosine synthesis, leading to elevated inosine levels (Figure 9C).

In summary, hepatic TKT deficiency not only prevents the entry of inosine-derived R5P into glycolysis but also promotes the synthesis of inosine from glucose-derived R5P, ultimately leading to elevated intracellular inosine levels.

### 3. TKT deficiency enhances mitochondrial function by promoting the inosine-PKA-CREB pathway

It has been reported that inosine promotes energy expenditure in adipose tissue through the PKA-CREB-UCP1 pathway.

Next, the inventors investigated the role of inosine in hepatocytes. Treatment of wild-type primary hepatocytes with inosine revealed that inosine activated the PKA-CREB signaling pathway but did not increase UCP1 protein expression (Figure 11A). Interestingly, increased activity of the PKA-CREB axis was observed in TKT^{flox/flox} Alb^{cre} hepatocytes, which was consistent with inosine accumulation resulting from TKT deficiency (Figure 10A). The inventors further investigated whether TKT deficiency affected mitochondrial morphology and function in hepatocytes.

TKT-deficient hepatocytes exhibited higher mitochondrial DNA content (Figure 11B), and higher mRNA and protein levels of mitochondrial genes such as ATP6, Mt-Co1/2/3, Mt-Nd1/2/3/4/5/6, Cytb, Ndufa12, and Tom20 (Figures 11C-E). Transmission electron microscopy of liver tissues revealed that TKT deficiency increased mitochondrial density, diameter, and surface area (Figures 10B-F). TKT-deficient hepatocytes showed higher mitochondrial membrane potential, ATP production capacity, and mitochondrial ROS levels (Figures 10G-I). Seahorse assay results demonstrated enhanced oxygen consumption rate (OCR) in TKT-deficient hepatocytes (Figure 10J).

The inventors found that inosine treatment promoted mitochondrial activity in hepatocytes, with a more pronounced effect in TKT-deficient hepatocytes. After inosine addition, OCR levels, ATP production capacity, and spare respiratory capacity were significantly upregulated in TKT-deficient hepatocytes (Figures 11G, H). Therefore, TKT deficiency promotes mitochondrial activity and sensitivity to inosine stimulation in hepatocytes, thereby improving hepatic lipid metabolism.

To determine whether inosine enhances hepatic mitochondrial function through the PKA-CREB pathway, hepatocytes were treated with the PKA inhibitor H89. As expected, H89 effectively reduced the levels of phosphorylated PKA substrates in both TKT^{flox/flox} Alb^{cre} and TKT^{+/+} Alb^{cre} primary hepatocytes (Figure 11F). Importantly, inosine failed to upregulate mitochondrial function in H89-treated primary hepatocytes (Figure 11I).

It has been reported that hyperinsulinemia interferes with mitochondrial function. The inventors found that insulin reduced intracellular inosine levels (Figure 10K) and inhibited mitochondrial activity in wild-type primary hepatocytes (Figure 10L). To investigate whether InsR and TKT play important roles in insulin-mediated reduction of inosine, primary hepatocytes were isolated from InsR^{flox/flox} Alb^{cre}, TKT^{flox/flox} Alb^{cre}, and control mice. It was found that insulin failed to affect inosine levels in InsR- or TKT-deficient hepatocytes (Figure 10M).

### 4. TKT deficiency promotes hepatocyte mitochondrial function by increasing phosphatidylcholine (PC) synthesis

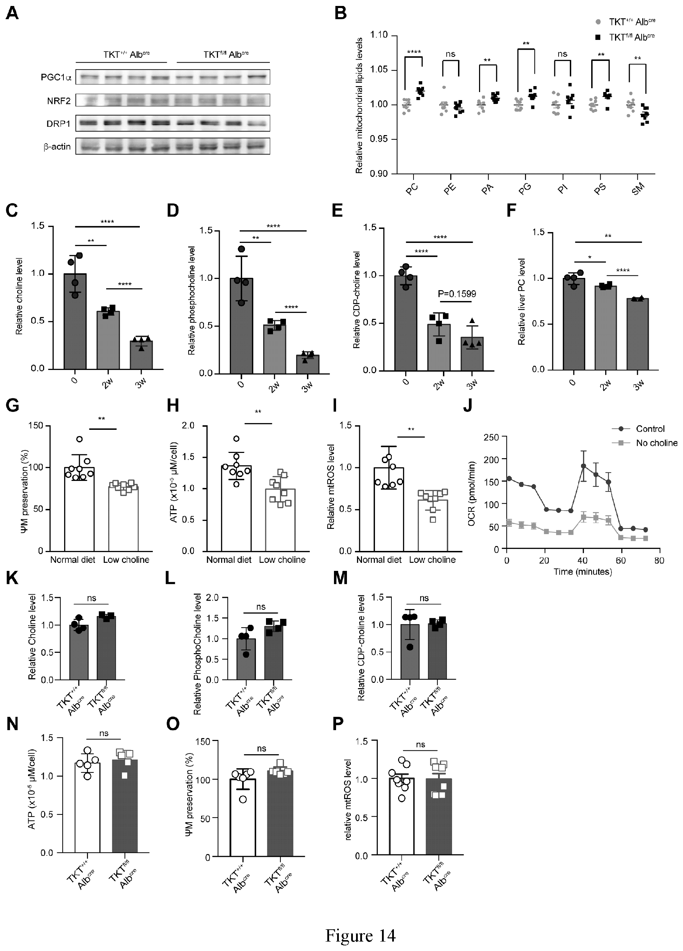

To investigate the regulatory role of mitochondrial morphological and functional changes induced by TKT deficiency in hepatocytes, the inventors compared the levels of PGC1α, NRF2, and DRP1, which regulate mitochondrial fission and fusion, in TKT^{flox/flox} Alb^{cre} and TKT^{+/+} Alb^{cre} primary hepatocytes. However, TKT deficiency did not alter the levels of PGC1α, NRF2, and DRP1 in hepatocytes (Figure 14A).

It has been reported that alterations in cellular lipid composition can affect mitochondrial morphology and function. Therefore, lipidomics analysis was performed on human liver samples (n = 48) to identify candidate lipids that are altered by TKT deficiency and may affect mitochondrial function. Approximately 700 lipids across 10 classes were identified. All lipids were divided into 15 modules using weighted gene co-expression network analysis (WGCNA) (Figure 12A), and correlation analysis between these modules and TKT protein levels was performed. Three lipid modules showed a negative correlation with TKT protein levels, with a Pearson correlation coefficient > 0.8 and p-value < 0.01 (Figure 12A).

After analyzing the central lipids of these 3 modules, six lipids were identified: PC, sphingomyelin (SM), N-acylethanolamine (NAE), ceramide (Cer), TG, and diacylglycerol (DG) (Figures 12B-D). PC is the most abundant phospholipid in the mitochondrial membrane and was the most significantly downregulated lipid in NAFLD patients (Figure 12E).

Furthermore, TKT deficiency in mouse hepatocytes led to PC upregulation (Figure 12G). Intracellular PC synthesis utilizes either the phosphatidylethanolamine (PE) methylation pathway or the CDP-choline pathway (Figure 12F), and PC is essential for mitochondrial function. The inventors' lipidomics results showed that mitochondrial PC levels, but not PE levels, were upregulated in TKT-deficient hepatocytes (Figure 14B), indicating that the PE methylation pathway, which relies on PE supply, was not perturbed. As the major pathway for intracellular PC synthesis, the CDP-choline pathway generates 60-70% of total intracellular PC. In addition to PC, the levels of CDP-choline pathway metabolites, including choline, phosphocholine, and CDP-choline, were all upregulated in TKT^{flox/flox} Alb^{cre} primary hepatocytes (Figure 12H), suggesting that TKT deficiency increases mitochondrial PC levels by promoting the CDP-choline pathway.

To investigate whether intracellular PC levels were critical for mitochondrial function in mouse hepatocytes, the inventors reduced dietary choline intake by 80% to limit PC biosynthesis. After 3 weeks of a low-choline diet, hepatic choline, phosphocholine, CDP-choline, and PC levels all decreased when compared with the normal diet group (Figures 14C-F). Importantly, low choline intake impaired the mitochondrial membrane potential, ATP production capacity, mitochondrial ROS, and OCR levels in hepatocytes (Figures 14G-J). Moreover, choline-restricted diets eliminated the difference in levels of CDP-choline pathway metabolites (Figures 12I and 14K-M) and mitochondrial activity (Figures 12J and 14N-P) between TKT^{flox/flox} Alb^{cre} and TKT^{+/+} Alb^{cre} primary hepatocytes.

These results indicate that hepatic TKT deficiency enhances mitochondrial activity and improves hepatic lipid metabolism by increasing intracellular PC synthesis.

### 5. Inosine stimulates PC synthesis by activating the PKA-CREB-ChoKβ axis in hepatocytes

Next, whether and how inosine regulates PC synthesis in hepatocytes was investigated. Inosine upregulated the levels of CDP-choline pathway metabolites in wild-type primary hepatocytes (Figure 12K). Choline kinase is a key enzyme in the CDP-choline pathway, catalyzing the first step of this pathway. Compared with the control group, the mRNA and protein levels of ChoKβ were increased in TKT^{flox/flox} Alb^{cre} primary hepatocytes (Figures 12L, M).

PROMO was utilized to predict potential transcription factors of ChoKβ, and it was found that CREB may regulate the transcriptional activity of ChoKβ. Importantly, the PKA inhibitor H89 eliminated inosine-induced CDP-choline pathway activity (Figure 12N).

These data indicate that, in TKT-deficient mouse hepatocytes, accumulation of inosine promotes intracellular PC synthesis and mitochondrial function by activating the PKA-CREB-ChoKβ pathway, thereby improving hepatic lipid metabolism.

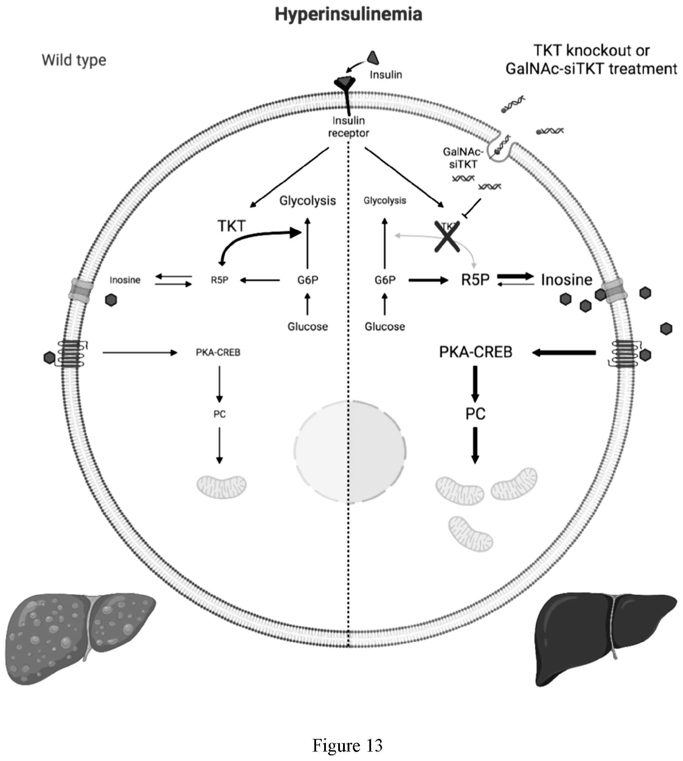

In summary, a high-fat diet induces hyperinsulinemia and promotes TKT expression through the insulin receptor (InsR)-CCAAT/enhancer-binding protein α (C/EBPα) pathway, thereby reducing intracellular inosine levels in hepatocytes. Conversely, TKT deficiency elevates hepatic inosine levels, and the accumulation of inosine promotes intracellular PC synthesis and mitochondrial function by activating the PKA-CREB-ChoKβ pathway (Figure 13).

### Example 5. Development and function of GalNAc-siRNA targeting hepatic TKT

### 1. Development of GalNAc-siRNA and its regulation of hepatic TKT

Methods such as targeted knockout require genomic modification and manipulation at the embryonic stage, which lack practicality for clinical treatment. To explore the possibility of targeting hepatic TKT for the treatment of NAFLD, the inventors performed screening and research analysis and obtained two siRNA reagents.
siTKT10 sequence:
   sense: CCGUGGACAUUGCUAACAUTT (SEQ ID NO: 49);
   antisense: AUGUUAGCAAUGUCCACGGTT (SEQ ID NO: 51).
siTKT16 sequence:
   sense: GCUGCAGAGAGUCUAAAGATT (SEQ ID NO: 50);
   antisense: UCUUUAGACUCUCUGCAGCTT (SEQ ID NO: 52).
   siNC sequence: scrambled sequence.

The 3' end of the siRNA sense strand was covalently conjugated to N-acetylgalactosamine (GalNAc), and solid-phase synthesis using a CPG support was employed as the preparation method.

The GalNAc moiety can be specifically recognized by the receptor ASGPR on the hepatocyte membrane, and the siRNA duplex is subsequently endocytosed into the cells. Therefore, the covalently conjugated product enables hepatocyte-targeted delivery of siRNA. Two GalNAc conjugates of siTKT, GalNAc-siTKT10 and GalNAc-siTKT16, were obtained, and GalNAc-siNC was prepared as a control.

The two GalNAc conjugates of siTKT efficiently knocked down TKT in mouse primary hepatocytes without lipid nanoparticle encapsulation (Figures 6A, B).

The inventors performed in vivo experiments in mice using GalNAc-siTKT16. Subcutaneous injection of GalNAc-siTKT16 reduced TKT protein levels in the liver but did not affect other tissues (Figures 6C-H).

The structure of GalNAc-siTKT16 is as follows:
AS (antisense):
   UmCfUmUmUmAfGmAmCmUmCmUmCmUfGmCfAmGmCmAmGmCmCm;
SS (sense):
   CmUmGmCmUmGmCfAmGfAfGfAmGmUmCmUmAmAmAmGmAm-L96.

Therefore, the GalNAc-siRNA targeting hepatic TKT exhibits a significant and specific downregulatory effect on TKT, thereby possessing the ability to effectively ameliorate NAFL and NASH.

### 2. Effect of GalNAc-siTKT on NAFL

The therapeutic effect of GalNAc-siTKT on NAFL was investigated at the animal level. After mice were fed with a HFD for 90 days, they received subcutaneous injections of GalNAc-siTKT on days 3, 7, 14, and 21 (Figure 5A), and were analyzed on day 30.

The results showed that GalNAc-siTKT significantly reduced TKT levels in the liver (Figure 5B), decreased hepatic lipid accumulation (Figure 5C), reduced serum and liver triglyceride and total cholesterol levels (Figures 5D-G), as well as serum ALT and AST levels (Figures 5H, I).

### 3. Therapeutic effect of GalNAc-siTKT on NASH

The preventive potential of GalNAc-siTKT against MCD-induced NASH in mice was further investigated. At the start of MCD feeding, a subcutaneous injection of GalNAc-siTKT (10 mg/kg) was administered, followed by additional injections on days 3, 7, 14, and 21 (Figure 5J).

On day 30, GalNAc-siTKT was found to reduce hepatic TKT levels (Figure 5K), alleviate hepatic steatosis, and ameliorate liver fibrosis (Figure 5L). GalNAc-siTKT intervention also reduced serum and liver triglyceride and total cholesterol levels (Figures 5M-P), as well as serum ALT and AST levels (Figures 5Q, R).

Furthermore, GalNAc-siTKT treatment reduced the levels of inflammation- and fibrosis-related genes but did not affect fatty acid synthesis genes (Figures 5S-U).

In summary, GalNAc-siTKT specifically targets TKT in hepatocytes and significantly ameliorates NAFL and NASH, providing a therapeutic strategy for the treatment of human NAFLD.

### Example 6. Screening and efficacy analysis of homologous sequences in human, mouse, and monkey

To obtain the siTKT sequence with the optimal knockdown effect, the inventors performed comprehensive sequence screening and experimental analysis of the homologous regions of the TKT gene in human, mouse, and monkey; full-genome sequences were also considered to exclude off-target effects. From a large number of candidate siRNAs, approximately 48 siRNA groups were obtained through primary screening (Table 1).

**Table 1**

| NO. | Sense (5-3) | SEQ ID NO: | NO. | Sense (5-3) | SEQ ID NO: |
|---|---|---|---|---|---|
| 52 | GCUCCAUCCAGGCCACCACUU | 1 | 780 | GCCAACAGCCAUCAUUGCCUU | 26 |
| 214 | GAGAUCAUGGCUGUCCUCUUU | 2 | 784 | ACAGCCAUCAUUGCCAAGAUU | 27 |
| 279 | CAAUGACCGCUUUGUGCUCUU | 3 | 785 | CAGCCAUCAUUGCCAAGACUU | 28 |
| 281 | AUGACCGCUUUGUGCUCUCUU | 4 | 787 | GCCAUCAUUGCCAAGACCUUU | 29 |
| 302 | AGGGCCAUGCAGCUCCCAUUU | 5 | 791 | UCAUUGCCAAGACCUUCAAUU | 30 |
| 327 | CGCGGUCUGGGCUGAAGCUUU | 6 | 794 | UUGCCAAGACCUUCAAGGGUU | 31 |
| 360 | GGCGGAGCUGCUGAACCUGUU | 7 | 800 | AGACCUUCAAGGGCCGAGGUU | 32 |
| 366 | GCUGCUGAACCUGAGGAAGUU | 8 | 803 | CCUUCAAGGGCCGAGGGAUUU | 33 |
| 375 | ACCUGAGGAAGAUCAGCUCUU | 9 | 1020 | GAUAGCCACCCGCAAGGCCUU | 34 |
| 435 | UGUGGCCACUGGCUCCCUGUU | 10 | 1059 | GCUGGGCCAUGCCAGUGACUU | 35 |
| 510 | GGCCAGCUACCGAGUCUAUUU | 11 | 1110 | UUCCACCUUCUCGGAGAUCUU | 36 |
| 519 | CCGAGUCUAUUGCUUGCUGUU | 12 | **1115** | **CCUUCUCGGAGAUCUUCAAUU** | 37 |
| 523 | GUCUAUUGCUUGCUGGGAGUU | 13 | 1292 | UCUCCGAGAGCAACAUCAAUU | 38 |
| 528 | UUGCUUGCUGGGAGACGGGUU | 14 | 1478 | UCUGCUUCAUCCGGACCAGUU | 39 |
| 666 | GCACCAGAUGGACAUCUACUU | 15 | 1526 | ACAAUGAGGACUUCCAGGUUU | 40 |
| 669 | CCAGAUGGACAUCUACCAGUU | 16 | 1542 | GGUCGGACAAGCCAAGGUGUU | 41 |
| 672 | GAUGGACAUCUACCAGAAGUU | 17 | 1549 | CAAGCCAAGGUGGUCCUGAUU | 42 |
| 719 | UCAUCGUGGAUGGACACAGUU | 18 | 1551 | AGCCAAGGUGGUCCUGAAGUU | 43 |
| 722 | UCGUGGAUGGACACAGCGUUU | 19 | 1552 | GCCAAGGUGGUCCUGAAGAUU | 44 |
| 729 | UGGACACAGCGUGGAGGAGUU | 20 | 1556 | AGGUGGUCCUGAAGAGCAAUU | 45 |
| 733 | CACAGCGUGGAGGAGCUGUUU | 21 | 1557 | GGUGGUCCUGAAGAGCAAGUU | 46 |
| 735 | CAGCGUGGAGGAGCUGUGCUU | 22 | 1737 | GGGCAGGAUCCUCACCGUGUU | 47 |
| 737 | GCGUGGAGGAGCUGUGCAAUU | 23 | 1741 | GGAUCCUCACCGUGGAGGAUU | 48 |
| 768 | GGCCAAGCACCAGCCAACAUU | 24 | | | |
| **773** | **AGCACCAGCCAACAGCCAUUU** | 25 | | | |

By comparing the mRNA and protein knockdown effects in mouse primary hepatocytes and the human LO2 cell line, sequences 773 and 1115 were selected as having the optimal knockdown effect (Figure 7A).

Sequences 773 (SEQ ID NO: 25) and 1115 (SEQ ID NO: 37) were modified with GalNAc to obtain GalNAc-siTKT 773 and GalNAc-siTKT 1115 for in vivo experiments in mice. GalNAc-siTKT 773 and GalNAc-siTKT 1115 were dissolved in PBS and administered to mice by subcutaneous injection at the back at a dose of 10 mg/kg.

After mice were fed a normal diet for 7 days, liver tissues were collected following euthanasia to assess TKT expression levels. It was found that the in vivo knockdown effect of sequence 1115 was superior to that of sequence 773 (Figures 7B and C).

Subsequently, an in vivo pharmacokinetic study of 1115 was conducted in mice. The results showed that following subcutaneous injection of GalNAc-siTKT at doses of 5 mg/kg and 10 mg/kg, respectively, the peak plasma concentration in mice occurred at approximately 60 minutes, and the in vivo half-life of the drug was approximately 4 hours (Figure 7D).

Therefore, the GalNAc-siTKT 773 and GalNAc-siTKT 1115, derived from homologous sequences in human, mouse, and monkey, exhibited excellent knockdown effects both in vitro and in vivo.

### Example 7. Further development of siTKT sequences with knockdown effect

### 1. siTKT

Based on an in-depth analysis of the TKT sequence, the inventors expanded the scale of sequence preparation and obtained a series of siRNAs, as shown in Table 2.

**Table 2. Unmodified siRNAs**

| **Duplex number** | **Seq ID** | **Sense 5'-3'** | **Seq ID** | **Antisense 5'-3'** |
|---|---|---|---|---|
| PPP24001 | 53 | GCUCCAUCCAGGCCACCAC | 168 | GUGGUGGCCUGGAUGGAGCUU |
| PPP24002 | 54 | GCCGCAGAGATCATGGCTG | 169 | CAGCCATGATCTCTGCGGCGC |
| PPP24003 | 55 | CCGCAGAGAUCAUGGCUGU | 170 | ACAGCCAUGAUCUCUGCGGUU |
| PPP24004 | 56 | CGCAGAGATCATGGCTGTC | 171 | GACAGCCATGATCTCTGCGGC |
| PPP24005 | 57 | GCAGAGATCATGGCTGTCC | 172 | GGACAGCCATGATCTCTGCGG |
| PPP24006 | 58 | CAGAGATCATGGCTGTCCT | 173 | AGGACAGCCATGATCTCTGCG |
| PPP24007 | 59 | AGAGATCATGGCTGTCCTC | 174 | GAGGACAGCCATGATCTCTGC |
| PPP24008 | 60 | GAGATCATGGCTGTCCTCT | 175 | AGAGGACAGCCATGATCTCTG |
| **PPP24009** | **61** | **GAUCAUGGCUGUCCUCUUU** | **176** | AAAGAGGACAGCCAUGAUCUU |
| PPP24010 | 62 | CATGGCTGTCCTCTTTTTC | 177 | GAAAAAGAGGACAGCCATGAT |
| **PPP24011** | **63** | **ATGGCTGTCCTCTTTTTCC** | **178** | GGAAAAAGAGGACAGCCATGA |
| PPP24012 | 64 | UGGCUGUCCUCUUUUUCCA | 179 | UGGAAAAAGAGGACAGCCAUU |
| PPP24013 | 65 | GGCTGTCCTCTTTTTCCAC | 180 | GTGGAAAAAGAGGACAGCCAT |
| PPP24014 | 66 | GCTGTCCTCTTTTTCCACA | 181 | TGTGGAAAAAGAGGACAGCCA |
| PPP24015 | 67 | CACCAUGCGCUACAAGUCC | 182 | GGACUUGUAGCGCAUGGUGUU |
| PPP24016 | 68 | CAUGCAGCUCCCAUCCUCU | 183 | AGAGGAUGGGAGCUGCAUGGC |
| PPP24017 | 69 | GTCCCGAAACAAGCTTTCA | 184 | TGAAAGCTTGTTTCGGGACCG |
| PPP24018 | 70 | GCAGCACCAGATGGACATC | 185 | GATGTCCATCTGGTGCTGCAG |
| PPP24019 | 71 | GCACCAGATGGACATCTAC | 186 | GTAGATGTCCATCTGGTGCTG |
| PPP24020 | 72 | GCCAACAGCCATCATTGCC | 187 | GGCAATGATGGCTGTTGGCTG |
| **PPP24021** | **73** | **CCAACAGCCATCATTGCCA** | **188** | TGGCAATGATGGCTGTTGGCT |
| **PPP24022** | **74** | **CAACAGCCAUCAUUGCCAA** | **189** | UUGGCAAUGAUGGCUGUUGUU |
| PPP24023 | 75 | AACAGCCATCATTGCCAAG | 190 | CTTGGCAATGATGGCTGTTGG |
| PPP24024 | 76 | ACAGCCATCATTGCCAAGA | 191 | TCTTGGCAATGATGGCTGTTG |
| PPP24025 | 77 | GCCAUCAUUGCCAAGACCU | 192 | AGGUCUUGGCAAUGAUGGCUU |
| **PPP24026** | **78** | **CCAUCAUUGCCAAGACCUU** | **193** | AAGGUCUUGGCAAUGAUGGCU |
| PPP24027 | 79 | CAGCCAGAUCCAGAGCAAA | 194 | UUUGCUCUGGAUCUGGCUGUU |
| PPP24028 | 80 | ACACCAAAAATTCCACCTT | 195 | AAGGTGGAATTTTTGGTGTCC |
| PPP24029 | 81 | CACCAAAAAUUCCACCUUC | 196 | GAAGGUGGAAUUUUUGGUGUU |
| PPP24030 | 82 | ACCAAAAATTCCACCTTCT | 197 | AGAAGGTGGAATTTTTGGTGT |
| PPP24031 | 83 | CCAAAAATTCCACCTTCTC | 198 | GAGAAGGTGGAATTTTTGGTG |
| PPP24032 | 84 | AUUCCACCUUCUCGGAGAU | 199 | AUCUCCGAGAAGGUGGAAUUU |
| PPP24033 | 85 | UCCACCUUCUCGGAGAUCU | 200 | AGAUCUCCGAGAAGGUGGAAU |
| PPP24034 | 86 | CACCTTCTCGGAGATCTTC | 201 | GAAGATCTCCGAGAAGGTGGA |
| PPP24035 | 87 | ACCTTCTCGGAGATCTTCA | 202 | TGAAGATCTCCGAGAAGGTGG |
| **PPP24036** | **88** | **CCUUCUCGGAGAUCUUCAA** | **203** | UUGAAGAUCUCCGAGAAGGUU |
| PPP24037 | 89 | CUUCUCGGAGAUCUUCAAA | 204 | UUUGAAGAUCUCCGAGAAGGU |
| PPP24038 | 90 | TTCTCGGAGATCTTCAAAA | 205 | TTTTGAAGATCTCCGAGAAGG |
| PPP24039 | 91 | TCTCGGAGATCTTCAAAAA | 206 | TTTTTGAAGATCTCCGAGAAG |
| PPP24040 | 92 | CAUCGAGUGCUACAUUGCU | 207 | AGCAAUGUAGCACUCGAUGAA |
| PPP24041 | 93 | ATCGAGTGCTACATTGCTG | 208 | CAGCAATGTAGCACTCGATGA |
| PPP24042 | 94 | TCGAGTGCTACATTGCTGA | 209 | TCAGCAATGTAGCACTCGATG |
| PPP24043 | 95 | TGAGCAGAACATGGTGAGC | 210 | GCTCACCATGTTCTGCTCAGC |
| PPP24044 | 96 | GAGCAGAACATGGTGAGCA | 211 | TGCTCACCATGTTCTGCTCAG |
| PPP24045 | 97 | AGCAGAACAUGGUGAGCAU | 212 | AUGCUCACCAUGUUCUGCUUU |
| PPP24046 | 98 | GCAGAACATGGTGAGCATC | 213 | GATGCTCACCATGTTCTGCTC |
| PPP24047 | 99 | TCTGCAGCACTTTTGCAGC | 214 | GCTGCAAAAGTGCTGCAGAAG |
| PPP24048 | 100 | CAGCACTTTTGCAGCCTTC | 215 | GAAGGCTGCAAAAGTGCTGCA |
| PPP24049 | 101 | AGCACTTTTGCAGCCTTCT | 216 | AGAAGGCTGCAAAAGTGCTGC |
| PPP24050 | 102 | GCACUUUUGCAGCCUUCUU | 217 | AAGAAGGCUGCAAAAGUGCUU |
| PPP24051 | 103 | CACTTTTGCAGCCTTCTTC | 218 | GAAGAAGGCTGCAAAAGTGCT |
| PPP24052 | 104 | ACUUUUGCAGCCUUCUUCA | 219 | UGAAGAAGGCUGCAAAAGUGC |
| PPP24053 | 105 | CTTTTGCAGCCTTCTTCAC | 220 | GTGAAGAAGGCTGCAAAAGTG |
| PPP24054 | 106 | TTTTGCAGCCTTCTTCACG | 221 | CGTGAAGAAGGCTGCAAAAGT |
| PPP24055 | 107 | GGCCTTTGACCAGATTCGC | 222 | GCGAATCTGGTCAAAGGCCCG |
| PPP24056 | 108 | GCCTTTGACCAGATTCGCA | 223 | TGCGAATCTGGTCAAAGGCCC |
| PPP24057 | 109 | CCUUUGACCAGAUUCGCAU | 224 | AUGCGAAUCUGGUCAAAGGUU |
| PPP24058 | 110 | CTTTGACCAGATTCGCATG | 225 | CATGCGAATCTGGTCAAAGGC |
| PPP24059 | 111 | CGCCATCTCCGAGAGCAAC | 226 | GTTGCTCTCGGAGATGGCGGC |
| PPP24060 | 112 | GCCATCTCCGAGAGCAACA | 227 | TGTTGCTCTCGGAGATGGCGG |
| PPP24061 | 113 | CCAUCUCCGAGAGCAACAU | 228 | AUGUUGCUCUCGGAGAUGGCG |
| PPP24062 | 114 | CATCTCCGAGAGCAACATC | 229 | GATGTTGCTCTCGGAGATGGC |
| PPP24063 | 115 | ATCTCCGAGAGCAACATCA | 230 | TGATGTTGCTCTCGGAGATGG |
| PPP24064 | 116 | UCUCCGAGAGCAACAUCAA | 231 | UUGAUGUUGCUCUCGGAGAUU |
| PPP24065 | 117 | CTCCGAGAGCAACATCAAC | 232 | GTTGATGTTGCTCTCGGAGAT |
| PPP24066 | 118 | TCCGAGAGCAACATCAACC | 233 | GGTTGATGTTGCTCTCGGAGA |
| PPP24067 | 119 | CCGAGAGCAACAUCAACCU | 234 | AGGUUGAUGUUGCUCUCGGAG |
| PPP24068 | 120 | AGAGCAACATCAACCTCTG | 235 | CAGAGGTTGATGTTGCTCTCG |
| PPP24069 | 121 | CCACTGCGGCGTTTCCATC | 236 | GATGGAAACGCCGCAGTGGGA |
| PPP24070 | 122 | CCTAGAAGATCTGGCTATG | 237 | CATAGCCAGATCTTCTAGGGC |
| PPP24071 | 123 | CUAGAAGAUCUGGCUAUGU | 238 | ACAUAGCCAGAUCUUCUAGGG |
| PPP24072 | 124 | TAGAAGATCTGGCTATGTT | 239 | AACATAGCCAGATCTTCTAGG |
| PPP24073 | 125 | AGAAGATCTGGCTATGTTT | 240 | AAACATAGCCAGATCTTCTAG |
| PPP24074 | 126 | TTCGGTCAGTCCCCACATC | 241 | GATGTGGGGACTGACCGAAAC |
| PPP24075 | 127 | TCGGTCAGTCCCCACATCA | 242 | TGATGTGGGGACTGACCGAAA |
| PPP24076 | 128 | CGGUCAGUCCCCACAUCAA | 243 | UUGAUGUGGGGACUGACCGAA |
| PPP24077 | 129 | GGTCAGTCCCCACATCAAC | 244 | GTTGATGTGGGGACTGACCGA |
| **PPP24078** | **130** | **GTCAGTCCCCACATCAACT** | **245** | AGTTGATGTGGGGACTGACCG |
| PPP24079 | 131 | TCAGTCCCCACATCAACTG | 246 | CAGTTGATGTGGGGACTGACC |
| PPP24080 | 132 | CAGUCCCCACAUCAACUGU | 247 | ACAGUUGAUGUGGGGACUGAC |
| PPP24081 | 133 | GUCCCCACAUCAACUGUCU | 248 | AGACAGUUGAUGUGGGGACUU |
| PPP24082 | 134 | CCCCACAUCAACUGUCUUU | 249 | AAAGACAGUUGAUGUGGGGAC |
| PPP24083 | 135 | CCCACATCAACTGTCTTTT | 250 | AAAAGACAGTTGATGTGGGGA |
| PPP24084 | 136 | CCACAUCAACUGUCUUUUA | 251 | UAAAAGACAGUUGAUGUGGGG |
| PPP24085 | 137 | CACATCAACTGTCTTTTAC | 252 | GTAAAAGACAGTTGATGTGGG |
| PPP24086 | 138 | ACATCAACTGTCTTTTACC | 253 | GGTAAAAGACAGTTGATGTGG |
| PPP24087 | 139 | CAUCAACUGUCUUUUACCC | 254 | GGGUAAAAGACAGUUGAUGUU |
| PPP24088 | 140 | TACAGAGAAGGCAGTGGAA | 255 | TTCCACTGCCTTCTCTGTAGC |
| PPP24089 | 141 | AGAAAATGCCATCATCTAT | 256 | ATAGATGATGGCATTTTCTGG |
| PPP24090 | 142 | GCCAAGGUGGUCCUGAAGA | 257 | UCUUCAGGACCACCUUGGCUU |
| PPP24091 | 143 | AGGUGGUCCUGAAGAGCAA | 258 | UUGCUCUUCAGGACCACCUUU |
| PPP24092 | 144 | GGUCCUGAAGAGCAAGGAU | 259 | AUCCUUGCUCUUCAGGACCAC |
| PPP24093 | 145 | ACTGCTGAAGAAAGAAAAG | 260 | CTTTTCTTTCTTCAGCAGTTC |
| PPP24094 | 146 | CCCTGGACAGAAAACTCAT | 261 | ATGAGTTTTCTGTCCAGGGGC |
| PPP24095 | 147 | CCUGGACAGAAAACUCAUU | 262 | AAUGAGUUUUCUGUCCAGGGG |
| PPP24096 | 148 | CTGGACAGAAAACTCATTC | 263 | GAATGAGTTTTCTGTCCAGGG |
| PPP24097 | 149 | TGGACAGAAAACTCATTCT | 264 | AGAATGAGTTTTCTGTCCAGG |
| PPP24098 | 150 | CACCGTGGAGGACCATTAT | 265 | ATAATGGTCCTCCACGGTGAG |
| PPP24099 | 151 | CCGUGGAGGACCAUUAUUA | 266 | UAAUAAUGGUCCUCCACGGUU |
| PPP24100 | 152 | TGGAGGACCATTATTATGA | 267 | TCATAATAATGGTCCTCCACG |
| PPP24101 | 153 | GCTGAGCTGCTGAAGATGT | 268 | ACATCTTCAGCAGCTCAGCCG |
| PPP24102 | 154 | GGGTCTATACATTCCTGAG | 269 | CTCAGGAATGTATAGACCCCC |
| PPP24103 | 155 | GAUUCUGGGAAAGGUGCUC | 270 | GAGCACCUUUCCCAGAAUCUU |
| PPP24104 | 156 | TCTGGGAAAGGTGCTCAAA | 271 | TTTGAGCACCTTTCCCAGAAT |
| PPP24105 | 157 | CTGGGAAAGGTGCTCAAAG | 272 | CTTTGAGCACCTTTCCCAGAA |
| PPP24106 | 158 | UGGGAAAGGUGCUCAAAGA | 273 | UCUUUGAGCACCUUUCCCAUU |
| PPP24107 | 159 | GGAAAGGUGCUCAAAGAUG | 274 | CAUCUUUGAGCACCUUUCCUU |
| PPP24108 | 160 | GAAAGGUGCUCAAAGAUGU | 275 | ACAUCUUUGAGCACCUUUCUU |
| PPP24109 | 161 | AAAGGTGCTCAAAGATGTA | 276 | TACATCTTTGAGCACCTTTCC |
| PPP24110 | 162 | AAGGUGCUCAAAGAUGUAC | 277 | GUACAUCUUUGAGCACCUUUU |
| PPP24111 | 163 | AGGUGCUCAAAGAUGUACU | 278 | AGUACAUCUUUGAGCACCUUU |
| PPP24112 | 164 | GGUGCUCAAAGAUGUACUG | 279 | CAGUACAUCUUUGAGCACCUU |
| PPP24113 | 165 | GTGCTCAAAGATGTACTGA | 280 | TCAGTACATCTTTGAGCACCT |
| PPP24114 | 166 | AAAGATGTACTGAGAGGAG | 281 | CTCCTCTCAGTACATCTTTGA |
| PPP24115 | 167 | TGAGAGGAGGGGTAAATAT | 282 | ATATTTACCCCTCCTCTCAGT |

Based on the sense and antisense strands of the siRNAs shown in Table 2, the inventors prepared modified sense and antisense strands using the methods described above, as shown in Figure 15.

The modifications for each nucleotide are described as follows:
The letter "m" indicates that the nucleotide immediately adjacent to the left of the letter "m" is a 2'-methoxy-modified nucleotide (Figure 16).

The letter "f" indicates that the nucleotide immediately adjacent to the left of the letter "f" is a 2'-fluoro-modified nucleotide (Figure 16).

The letter "s" indicates that the linkage between the two nucleotides adjacent to the left and right of the letter "s" or between the linked nucleotide and the GalNAc is a phosphorothioate linkage (Figure 16).

### 2. Inhibitory effect of modified siTKT on the target gene TKT

At 24 hours prior to transfection, Hep3B cells were seeded in 96-well plates at approximately 20,000 cells/well, with 100 µL of culture medium per well. At the time of transfection, samples were transfected using Lipofectamine RNAiMAX (Invitrogen, 13778150) according to the product manual, at a single concentration with a final concentration of 2.5 nM. At 24 hours after treatment, total cellular RNA was extracted using a high-throughput cell RNA extraction kit (FG0417-L/FG0418-XL, magnetic bead method). RNA reverse transcription (ThermoFisher, AK16225) and real-time quantitative PCR (ThermoFisher, A25778) were performed to determine the mRNA level of human TKT, which was normalized to that of the HPRT internal control gene.

The modified sequences were evaluated for their inhibition of target gene TKT mRNA expression using Hep3B cells at a single concentration. As shown in Table 3, based on the sequence characteristics of the target TKT mRNA, sequences with significantly superior knockdown of TKT mRNA were screened out. At a concentration of 2.5 nM, multiple sequences were able to reduce target gene TKT mRNA expression levels to below 0.1, with the lowest being reduced to 0.03.

Based on the inhibitory efficiency of the sequences in Table 3 at a single concentration, several sequences were selected for multi-concentration evaluation of their inhibitory effect on target gene TKT mRNA expression. The IC₅₀ value corresponds to the concentration of siRNA at which the remaining percentage of TKT mRNA expression is 50%, and the maximum inhibition percentage corresponds to the maximum extent to which TKT mRNA expression can be inhibited across multiple concentrations.

**Table 3**

| **Duplex number** | **Residual value of target gene TKT mRNA expression (mean)** |
|---|---|
| | **2.5nM** |
| MPPP24001 | 0.17 |
| MPPP24002 | 0.08 |
| MPPP24003 | 0.13 |
| MPPP24004 | 0.09 |
| MPPP24005 | 0.18 |
| MPPP24006 | 0.07 |
| MPPP24007 | 0.08 |
| MPPP24008 | 0.42 |
| **MPPP24009** | **0.06** |
| MPPP24010 | 0.53 |
| **MPPP24011** | **0.05** |
| MPPP24012 | 0.11 |
| MPPP24013 | 0.06 |
| MPPP24014 | 0.07 |
| MPPP24015 | 0.18 |
| MPPP24016 | 0.04 |
| MPPP24017 | 0.03 |
| MPPP24018 | 0.08 |
| MPPP24019 | 0.07 |
| MPPP24020 | 0.70 |
| **MPPP24021** | **0.07** |
| **MPPP24022** | **0.05** |
| MPPP24023 | 0.82 |
| MPPP24024 | 0.33 |
| MPPP24025 | 0.60 |
| **MPPP24026** | **0.16** |
| MPPP24027 | 0.06 |
| MPPP24028 | 0.03 |
| MPPP24029 | 0.13 |
| MPPP24030 | 0.03 |
| MPPP24031 | 0.06 |
| MPPP24032 | 0.18 |
| MPPP24033 | 0.17 |
| MPPP24034 | 0.10 |
| MPPP24035 | 0.04 |
| **MPPP24036** | **0.07** |
| MPPP24037 | 0.29 |
| MPPP24038 | 0.05 |
| MPPP24039 | 0.02 |
| MPPP24040 | 0.08 |
| MPPP24041 | 0.06 |
| MPPP24042 | 0.05 |
| MPPP24043 | 0.03 |
| MPPP24044 | 0.15 |
| MPPP24045 | 0.11 |
| MPPP24046 | 0.06 |
| MPPP24047 | 0.09 |
| MPPP24048 | 0.09 |
| MPPP24049 | 0.05 |
| MPPP24050 | 0.08 |
| MPPP24051 | 0.07 |
| MPPP24052 | 0.08 |
| MPPP24053 | 0.07 |
| MPPP24054 | 0.12 |
| MPPP24055 | 0.07 |
| MPPP24056 | 0.04 |
| MPPP24057 | 0.09 |
| MPPP24058 | 0.06 |
| MPPP24059 | 0.12 |
| MPPP24060 | 0.13 |
| MPPP24061 | 0.10 |
| MPPP24062 | 0.06 |
| MPPP24063 | 0.14 |
| MPPP24064 | 0.13 |
| MPPP24065 | 0.05 |
| MPPP24066 | 0.07 |
| MPPP24067 | 0.06 |
| MPPP24068 | 0.05 |
| MPPP24069 | 0.04 |
| MPPP24070 | 0.08 |
| MPPP24071 | 0.08 |
| MPPP24072 | 0.04 |
| MPPP24073 | 0.05 |
| MPPP24074 | 0.09 |
| MPPP24075 | 0.34 |
| MPPP24076 | 0.06 |
| MPPP24077 | 0.14 |
| **MPPP24078** | **0.04** |
| MPPP24079 | 0.10 |
| MPPP24080 | 0.15 |
| MPPP24081 | 0.26 |
| MPPP24082 | 0.58 |
| MPPP24083 | 0.03 |
| MPPP24084 | 0.08 |
| MPPP24085 | 0.03 |
| MPPP24086 | 0.03 |
| MPPP24087 | 0.19 |
| MPPP24088 | 0.05 |
| MPPP24089 | 0.04 |
| MPPP24090 | 1.12 |
| MPPP24091 | 0.16 |
| MPPP24092 | 0.29 |
| MPPP24093 | 0.04 |
| MPPP24094 | 0.04 |
| MPPP24095 | 0.09 |
| MPPP24096 | 0.07 |
| MPPP24097 | 0.05 |
| MPPP24098 | 0.08 |
| MPPP24099 | 0.10 |
| MPPP24100 | 0.05 |
| MPPP24101 | 0.05 |
| MPPP24102 | 0.06 |
| MPPP24103 | 0.32 |
| MPPP24104 | 0.05 |
| MPPP24105 | 0.09 |
| MPPP24106 | 0.09 |
| MPPP24107 | 0.21 |
| MPPP24108 | 0.08 |
| MPPP24109 | 0.06 |
| MPPP24110 | 0.06 |
| MPPP24111 | 0.05 |
| MPPP24112 | 0.05 |
| MPPP24113 | 0.08 |
| MPPP24114 | 0.09 |
| MPPP24115 | 0.07 |
| 1115 sequence | 0.33 |

At 24 hours prior to transfection, Hep3B cells were seeded in 96-well plates at approximately 20,000 cells/well, with 100 µL of culture medium per well. At the time of transfection, samples were transfected using Lipofectamine RNAiMAX (Invitrogen, 13778150) according to the product manual. The gradient final concentrations for multi-concentration sample transfection were 5, 1, 0.2, 0.04, 0.008, 0.0016, and 0.00032 nM. At 24 hours after treatment, total cellular RNA was extracted using a high-throughput cell RNA extraction kit (FG0417-L/FG0418-XL, magnetic bead method). RNA reverse transcription (ThermoFisher, AK16225) and real-time quantitative PCR (ThermoFisher, A25778) were performed to determine the mRNA level of human TKT, which was normalized to that of the HPRT internal control gene.

The IC₅₀ values and maximum inhibition percentages of different sequences were calculated by nonlinear fitting of the residual TKT mRNA values obtained after treatment with the above-mentioned different concentrations of siRNA. The IC₅₀ value corresponds to the concentration of siRNA at which the remaining percentage of TKT mRNA expression is 50%, and the maximum inhibition percentage corresponds to the maximum extent to which TKT mRNA expression can be inhibited across multiple concentrations. The results of multi-concentration screening of modified sequences for inhibition of target gene TKT mRNA expression in Hep3B cells, in terms of IC₅₀ values, are shown in Table 4.

The results of multi-concentration screening of modified sequences for inhibition of target gene TKT mRNA expression in Hep3B cells are shown in Table 4.

**Table 4**

| **Duplex number** | **IC₅₀ (nM)** | **Maximum inhibition percentage (100%)** |
|---|---|---|
| MPPP24001 | 0.208 | 87.86 |
| MPPP24007 | 0.101 | 92.58 |
| **MPPP24009** | **0.008** | **95.56** |
| **MPPP24011** | **0.008** | **96.23** |
| MPPP24016 | 0.025 | 94.97 |
| MPPP24017 | 0.021 | 94.95 |
| MPPP24018 | 0.158 | 92.42 |
| MPPP24019 | 0.045 | 92.95 |
| **MPPP24021** | **0.074** | **93.44** |
| **MPPP24022** | **0.095** | **94.65** |
| **MPPP24026** | **0.128** | **94.78** |
| MPPP24027 | 0.087 | 92.98 |
| MPPP24028 | 0.067 | 96.30 |
| MPPP24030 | 0.036 | 96.02 |
| MPPP24031 | 0.128 | 91.94 |
| MPPP24035 | 0.127 | 93.69 |
| **MPPP24036** | **0.008** | **96.84** |
| MPPP24038 | 0.099 | 92.22 |
| MPPP24039 | 0.032 | 96.14 |
| MPPP24040 | 0.051 | 95.71 |
| MPPP24043 | 0.026 | 95.38 |
| MPPP24045 | 0.044 | 96.63 |
| MPPP24052 | 0.023 | 92.02 |
| MPPP24055 | 0.135 | 90.62 |
| MPPP24056 | 0.109 | 93.04 |
| MPPP24058 | 0.067 | 93.71 |
| MPPP24061 | 0.040 | 95.56 |
| MPPP24062 | 0.033 | 92.24 |
| MPPP24067 | 0.238 | 95.74 |
| MPPP24068 | 0.039 | 95.69 |
| MPPP24069 | 0.018 | 95.80 |
| MPPP24072 | 0.021 | 95.52 |
| **MPPP24078** | **0.006** | **96.83** |
| MPPP24085 | 0.013 | 96.59 |
| MPPP24088 | 0.057 | 94.96 |
| MPPP24089 | 0.033 | 97.09 |
| MPPP24091 | 0.179 | 94.55 |
| MPPP24093 | 0.054 | 95.09 |
| MPPP24094 | 0.024 | 96.76 |
| MPPP24098 | 0.208 | 90.82 |
| MPPP24099 | 0.061 | 94.09 |
| MPPP24100 | 0.113 | 94.46 |
| MPPP24101 | 0.055 | 95.15 |
| MPPP24102 | 0.453 | 84.75 |
| MPPP24104 | 0.012 | 93.54 |
| MPPP24112 | 0.084 | 91.56 |
| MPPP24114 | 0.381 | 84.19 |
| MPPP24115 | 0.088 | 88.69 |

Based on the above, the inventors further identified advantageous sequence regions suitable for designing siTKT, as well as advantageous inhibition analyses, including:
duplex numbers PPP24074 to PPP24087, among which PPP24078 is relatively more preferred; as well as their corresponding modified sequences and conjugated molecules;
duplex numbers PPP24002 to PPP24014, among which PPP24009 and PPP24011 are relatively more preferred; as well as their corresponding modified sequences and conjugated molecules;
duplex numbers PPP24032 to PPP24039, among which PPP24036 is relatively more preferred; as well as their corresponding modified sequences and conjugated molecules; and
duplex numbers PPP24020 to PPP24026, among which PPP24021, PPP24022, and PPP24026 are relatively more preferred; as well as their corresponding modified sequences and conjugated molecules.

The above-described Examples merely express several implementation modes of the present disclosure, and their descriptions are relatively specific and detailed, but they should not be construed as limiting the patent scope of the present disclosure. It should be noted that, for those of ordinary skill in the art, several variations and improvements can be made without departing from the concept of the present disclosure, and these all fall within the protection scope of the present disclosure. Therefore, the protection scope of the patent of the present disclosure should be defined by the appended claims.

## Claims

1. Use of a downregulator of transketolase (TKT) or an insulin receptor (InsR)-CCAAT/enhancer-binding protein α (C/EBPα)-TKT signaling pathway comprising TKT in the preparation of a pharmaceutical composition for alleviating or treating metabolism-associated fatty liver disease.

2. The use according to claim 1, **characterized in that** the downregulator comprises a downregulator selected from: a downregulator of TKT, a downregulator of the C/EBPα-TKT interaction, or a downregulator of the InsR-C/EBPα interaction;
preferably, the downregulator of TKT comprises: an agent that silences, knocks down, or knocks out the TKT gene, or an agent that inhibits TKT protein activity; more preferably, the downregulator of TKT comprises: an interfering molecule that specifically interferes with TKT gene expression, a CRISPR gene editing reagent targeting the TKT gene, a homologous recombination reagent, or a site-directed mutagenesis reagent, wherein the reagent causes TKT to undergo a loss-of-function mutation;
preferably, the downregulator of the C/EBPα-TKT interaction comprises: an agent that weakens the binding of C/EBPα to the TKT promoter, an agent that silences, knocks down, or knocks out the C/EBPα gene, or an agent that inhibits C/EBPα protein activity; more preferably, the downregulator of the C/EBPα-TKT interaction comprises: an agent that reduces the phosphorylation level of C/EBPα protein, an interfering molecule that specifically interferes with C/EBPα gene expression, a CRISPR gene editing reagent targeting the C/EBPα gene, a homologous recombination reagent, or a site-directed mutagenesis reagent, wherein the reagent causes C/EBPα to undergo a loss-of-function mutation; or
preferably, the downregulator of the InsR-C/EBPα interaction comprises: an agent that silences, knocks down, or knocks out the InsR gene, an agent that inhibits InsR protein activity, or an agent that weakens insulin binding to InsR; more preferably, the downregulator of the InsR-C/EBPα interaction comprises: an interfering molecule that specifically interferes with InsR gene expression, a CRISPR gene editing reagent targeting the InsR gene, a homologous recombination reagent, or a site-directed mutagenesis reagent, wherein the reagent causes InsR to undergo a loss-of-function mutation.

3. The use according to claim 2, **characterized in that** the downregulator is an interfering reagent that silences the TKT gene; preferably, the interfering reagent is an siRNA; preferably, the siRNA is an siRNA having a nucleotide sequence as shown in any one of SEQ ID NO: 1 to SEQ ID NO: 50, and SEQ ID NO: 53 to SEQ ID NO: 167; more preferably, the siRNA is an siRNA having a nucleotide sequence as shown in any one of SEQ ID NO: 37, SEQ ID NO: 25, SEQ ID NO: 50, SEQ ID NO: 49, SEQ ID NO: 61, SEQ ID NO: 63, SEQ ID NO: 73, SEQ ID NO: 74, SEQ ID NO: 78, SEQ ID NO: 88, or SEQ ID NO: 130; or
the downregulator is a homologous recombination reagent that knocks down or knocks out the TKT gene, which causes the gene coding to be terminated; or
the downregulator is a homologous recombination reagent that knocks down or knocks out the InsR gene, which causes the gene coding to be terminated.

4. The use according to claim 2, **characterized in that** the siRNA is formulated as an siRNA formulation for hepatocyte-targeted delivery; preferably, the siRNA is linked to a targeting ligand that binds to asialoglycoprotein receptor; the targeting ligand comprises: N-acetylgalactosamine, N-trifluoroacetylgalactosamine, N-propionylgalactosamine, N-n-butyrylgalactosamine or N-isobutyrylgalactosamine.

5. The use according to claim 1, **characterized in that** the metabolism-associated fatty liver disease comprises: nonalcoholic fatty liver disease, nonalcoholic steatohepatitis; or
the downregulator of TKT or the InsR-C/EBPα-TKT signaling pathway comprising TKT increases inosine levels and enhances mitochondrial function, thereby improving hepatic lipid metabolism and alleviating or treating metabolism-associated fatty liver disease; preferably, the downregulator prevents inosine-derived R5P from entering glycolysis, promotes the synthesis of inosine from glucose-derived R5P, elevates intracellular inosine levels, enhances mitochondrial function, improves hepatic lipid metabolism, and alleviates or treats metabolism-associated fatty liver disease; preferably, increasing phosphatidylcholine synthesis is mediated by the CDP-choline pathway.

6. An siRNA or an siRNA formulation for alleviating or treating metabolism-associated fatty liver disease, wherein the siRNA comprises an siRNA having a nucleotide sequence as shown in any one of SEQ ID NO: 1 to SEQ ID NO: 50, and SEQ ID NO: 53 to SEQ ID NO: 167; preferably, the siRNA is an siRNA having a nucleotide sequence as shown in any one of SEQ ID NO: 37, SEQ ID NO: 25, SEQ ID NO: 50, SEQ ID NO: 49, SEQ ID NO: 61, SEQ ID NO: 63, SEQ ID NO: 73, SEQ ID NO: 74, SEQ ID NO: 78, SEQ ID NO: 88, or SEQ ID NO: 130.

7. The siRNA or siRNA formulation according to claim 6, **characterized in that** the siRNA formulation is a stabilized siRNA formulation for hepatocyte-targeted delivery; preferably, the siRNA is linked to a targeting ligand that binds to the asialoglycoprotein receptor; the targeting ligand comprises: N-acetylgalactosamine, N-trifluoroacetylgalactosamine, N-propionylgalactosamine, N-butyrylgalactosamine, or N-isobutyrylgalactosamine.

8. The siRNA or siRNA formulation according to claim 6 or 7, **characterized in that** in the siRNA, the nucleotides in the sense strand and the antisense strand comprise modifications selected from the group consisting of: 2'-methoxy modification, 2'-fluoro modification; or, between adjacent nucleotides or between a nucleotide and a targeting ligand, comprise a phosphorothioate linkage.

9. The siRNA or siRNA formulation according to claim 8, **characterized in that** in the sense strand of the siRNA, from the 5' end, there are phosphorothioate linkages between the 1st and 2nd nucleotides and between the 2nd and 3rd nucleotides; in the corresponding antisense strand of the siRNA, from the 5' end, there is a phosphorothioate linkage between the 1st and 2nd nucleotides; and from the 3' end, there are phosphorothioate linkages between the 1st and 2nd nucleotides and between the 2nd and 3rd nucleotides;
preferably, in the sense strand of the siRNA, from the 5' end, the nucleotides at positions 1, 2, 3, 4, 6, and 10-19 are 2'-methoxy modified; in the corresponding antisense strand of the siRNA, from the 5' end, the nucleotides at positions 1, 3, 4, 5, 7, 10-13, 15, and 17-21 are 2'-methoxy modified;
preferably, in the sense strand of the siRNA, from the 5' end, the nucleotides at positions 5, 7, 8, and 9 are 2'-fluoro modified; in the corresponding antisense strand of the siRNA, the nucleotides at positions 2, 6, 8, 9, 14, and 16 are 2'-fluoro modified.

10. A pharmaceutical composition or kit for alleviating or treating metabolism-associated fatty liver disease, comprising the siRNA or siRNA formulation for alleviating or treating metabolism-associated fatty liver disease according to any one of claims 6 to 8, and a pharmaceutically acceptable carrier or excipient.

11. Use of a InsR-C/EBPα-TKT signaling pathway in screening a substance for alleviating or treating metabolism-associated fatty liver disease.

12. A method for screening a substance for alleviating or treating metabolism-associated fatty liver disease, comprising:
(1) contacting a candidate substance with a system comprising the InsR-C/EBPα-TKT signaling pathway;
(2) screening for a substance that downregulates the InsR-C/EBPα-TKT signaling pathway, wherein the substance is a substance useful for alleviating or treating metabolism-associated fatty liver disease; wherein the downregulating comprises: downregulating TKT, downregulating the C/EBPα-TKT interaction, and downregulating the insulin/InsR-C/EBPα interaction.

13. The method according to claim 12, **characterized in that** the step (1) comprises: adding a candidate substance to a system comprising the InsR-C/EBPα-TKT signaling pathway;
the step (2) comprises: detecting changes in each protein or an encoding gene thereof in the InsR-C/EBPα-TKT signaling pathway, and comparing with a control group, wherein the control group is a system comprising the InsR-C/EBPα-TKT signaling pathway without adding the candidate substance; if the candidate substance downregulates TKT, downregulates the C/EBPα-TKT interaction, or downregulates the insulin/InsR-C/EBPα interaction, then the candidate substance is a substance useful for alleviating or treating metabolism-associated fatty liver disease.

14. Use of a hepatic TKT protein or an encoding gene thereof in the preparation of a diagnostic reagent, wherein the diagnostic reagent is used for diagnosing or prognosing metabolism-associated fatty liver disease; preferably, the diagnosing or prognosing comprises: determining the occurrence or progression of metabolism-associated fatty liver disease, or determining whether it is suitable for applying a treatment regimen that uses "a downregulator of TKT or the InsR-C/EBPα-TKT signaling pathway comprising TKT" for treatment, based on the expression status of the hepatic TKT protein; if the TKT protein is highly expressed, then the treatment regimen is applicable.
